# EUROPÄISCHE PATENTSCHRIFT

(11) **EP 2 758 382 B1**
(45) Veröffentlichungstag und Bekanntmachung des Hinweises auf die Patenterteilung: **09.11.2016**
(21) Anmeldenummer: 12762269.4
(22) Anmeldetag: 18.09.2012
(51) Int. Cl.: C07D 277/48, C07D 417/12, A61K 31/426, A61K 8/49, A61P 17/00, A61Q 19/02

(54) **HETEROCYCLOCARBONYLAMINOTHIAZOLE, SIE ENTHALTENDE KOSMETISCHE ODER DERMATOLOGISCHE ZUBEREITUNGEN UND DEREN VERWENDUNG ZUR BEKÀMPFUNG UND PROPHYLAXE UNERWÜNSCHTER PIGMENTIERUNG DER HAUT**
HETEROCYCLOCARBONYLAMINOTHIAZOLES, COSMETIC AND DERMATOLOGICAL PREPARATIONS CONTAINING THEM, AND THEIR USE FOR THE TREATMENT AND PROPHYLAXIS OF UNWANTED PIGMENTATION OF THE SKIN
HÉTÉROCYCLOCARBONYLAMINOTHIAZOLES, PRÉPARATIONS COSMÉTIQUES OU DERMATOLOGIQUES LES COMPRENANT, ET LEUR UTILISATION POUR LE TRAITEMENT ET LA PRÉVENTION DE LA PIGMENTATION INDÉSIRABLE DE LA PEAU

(30) Priorität: 23.09.2011 DE 102011083283
(43) Veröffentlichungstag der Anmeldung: 30.07.2014
(73) Patentinhaber: Beiersdorf AG, 20253 Hamburg (DE)
(72) Erfinder: KOLBE, Ludger, 21255 Dohren (DE); SCHERNER, Cathrin, 22844 Norderstedt (DE)
(86) Internationale Anmeldenummer: PCT/EP2012/068376
(87) Internationale Veröffentlichungsnummer: WO 2013/041537

(56) Entgegenhaltungen:
- EP-A1- 0 519 449
- EP-A1- 1 649 852
- WO-A2-2011/117034
- PRAKASHA, K. C. ET AL: "Design, synthesis and antimicrobial screening of amino acids conjugated 2-amino-4-arylthiazole derivatives", INTERNATIONAL JOURNAL OF PHARMACY AND PHARMACEUTICAL SCIENCES, CODEN: IJPPKB; ISSN: 0975-1491 URL: HTTP://WWW.IJPPSJOURNAL.COM/VOL3SUPPL3/215 3.PDF, Bd. 3, Nr. SUPPL 3, Mai 2011 (2011-05), Seiten 120-125, XP002687395,
- GERMANAS ET AL: "Discovery of small-molecule inhibitors of tyrosinase", BIOORGANIC & MEDICINAL CHEMISTRY LETTERS, PERGAMON, ELSEVIER SCIENCE, GB, Bd. 17, Nr. 24, 12. Oktober 2007 (2007-10-12), Seiten 6871-6875, XP022339589, ISSN: 0960-894X, DOI: 10.1016/J.BMCL.2007.10.014 in der Anmeldung erwähnt

## Beschreibung

Die vorliegende Erfindung betrifft neue Heteroalkylamidothiazole, kosmetische oder dermatologische Zubereitungen mit einem Gehalt an einem oder mehreren solcher Heteroalkylamidothiazole und die Verwendung solcher Heteroalkylamidothiazole bzw. Zubereitungen, solche Heteroalkylamidothiazole enthaltend, zur Bekämpfung und Prophylaxe unerwünschter Pigmentierung der Haut.

Für die Pigmentierung der Haut verantwortlich sind die Melanozyten, welche in der untersten Schicht der Epidermis, dem Stratum basale, neben den Basalzellen als - je nach Hauttyp entweder vereinzelt oder aber mehr oder weniger gehäuft auftretende - pigmentbildende Zellen vorzufinden sind.

Melanozyten enthalten als charakteristische Zellorganellen Melanosomen, in denen das Melanin gebildet wird. Unter anderem bei Anregung durch UV-Strahlung wird verstärkt Melanin gebildet. Dieses wird über die lebenden Schichten der Epidermis (Keratinozyten) letztlich in die Hornschicht (Corneozyten) transportiert und ruft eine mehr oder weniger ausgeprägte bräunliche bis braun-schwarze Hautfarbe hervor.

Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. DHICA- und DHI-Melanin entstehen über die gemeinsamen Zwischenstufen Dopachinon und Dopachrom. Letzteres wird, teilweise unter Beteiligung weiterer Enzyme, entweder in Indol-5,6-Chinon-Carbonsäure oder in Indol-5,6-Chinon umgesetzt, woraus die beiden genannten Eumelanine entstehen.

Die Entstehung von Phäomelanin läuft unter anderem über die Zwischenprodukte Dopachinon und Cysteinyldopa. Gesteuert wird die Expression der Melanin-synthetisierenden Enzyme durch einen spezifischen Transkriptionsfaktor (microphthalmia- associated transcription factor, MITF). Neben den beschriebenen enzymatischen Prozessen der Melanin-Synthese sind in den Melanosomen noch weitere Proteine für die Melanogenese von Bedeutung. Eine wichtige Rolle scheint hier dem sogenannten p-Protein zuzukommen, wobei die exakte Funktion noch unklar ist.

Neben dem zuvor beschriebenen Prozeß der Melanin-Synthese in den Melanozyten, ist bei der Pigmentierung der Haut auch der Transfer der Melanosomen, deren Verbleib in der Epidermis sowie deren Abbau und der Abbau des Melanins von entscheidender Bedeutung. Es konnte gezeigt werden daß für den Transport der Melanosomen aus den Melanozyten in die Keratinozyten der PAR-2-Rezeptor bedeutsam ist (M. Seiberg et al., 2000, J. Cell. Sci., 113:3093-101).

Ferner haben Größe und Form der Melanosomen Einfluß auf ihre lichtstreuenden Eigenschaften und somit das farbliche Erscheinungsbild der Haut. So findet man bei Schwarzafrikanern verstärkt große spheroidale, einzeln vorliegende Melanosomen, während man bei Kaukasiern eher kleinere, in Gruppen vorkommende Melanosomen vorfindet.

Probleme mit Hyperpigmentierung der Haut haben vielfältige Ursachen bzw. sind Begleiterscheinungen vieler biologischer Vorgänge, z.B. UV-Strahlung (z.B. Sommersprossen, *Ephelides*), genetische Disposition, Fehlpigmentierung der Haut bei der Wundheilung bzw. - vernarbung (postinflammatorische Hyperpigmentierung) oder der Hautalterung (z.B. *Lentigines seniles*).

Nach entzündlichen Reaktionen reagiert das Pigmentierungssystem der Haut mit teilweise entgegengesetzten Reaktionen. Es kann sowohl zu postinflammatorischen Hyper- wie auch Hypopigmentierungen kommen. Postinflammatorische Hypomelanosen treten u. a. häufig in Verbindung mit Atopie, Lupus erythematosus und Psoriasis auf. Die unterschiedlichen Reaktionsformen des Pigmentierungssystems der menschlichen Haut in Folge entzündlicher Erscheinungen sind nur sehr unvollständig verstanden.

Probleme mit postinflammatorischer Hyperpigmentierung treten häufig bei dunkleren Hauttypen auf. Insbesondere bei männlichen Farbigen ist das Problem der *Pseudofollikulitis barbae* bekannt, das mit kosmetisch unerwünschten Fehlpigmentierung einhergeht bzw. diese nach sich zieht. Auch Formen von Melasma, welche insbesondere bei Frauen asiatischer Zugehörigkeit im Gesicht und im Dekolleté-Bereich auftreten, sowie verschiedene Formen der unregelmäßigen Pigmentierung der Haut werden zu den postinflammatorischen Hyperpigmentierungen gezählt. Ferner werden auch dunkle Augenringe als eine Form postinflammatorischen Hyperpigmentierungen angesehen, wobei die zugrunde liegende Entzündung meist subklinisch abläuft.

In vielen Fällen wird derartige postinflammatorische Fehlpigmentierung durch Einwirkung von Sonnenlicht (UV-Licht) noch verstärkt, ohne daß es zu einer UV-induzierten Entzündung (Sonnenbrand) kommt.

Es sind Wirkstoffe und Zubereitungen bekannt, welche der Hautpigmentierung entgegenwirken. Im praktischen Gebrauch sind im wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Von Albert Kligman et al. wurde eine sogenannte "Triformula" entwickelt, die eine Kombination aus 0.1% Tretinoin, 5.0% Hydrochinon, 0.1% Dexamethason darstellt (A. Kligman, 1975, Arch. Dermatol., 111:40-48). Allerdings ist auch diese Formulierung wegen möglicher irreversibler Veränderungen im Pigmentierungssystem der Haut sehr umstritten.

Ferner finden hautschälende Methoden (chemische und mechanische "Peelings") Anwendung, die jedoch häufig entzündliche Reaktionen nach sich ziehen und aufgrund danach eintretender postinflammatorischer Hyperpigmentierungen sogar zu stärkerer statt verminderter Pigmentierung führen können. All diese gängigen Verfahren, die auch zur Behandlung von postinflammatorischen Hypergigmentierungen angewendet werden, zeichnen sich durch entscheidende Nebenwirkungen aus.

Weiterhin sind diverse andere Substanzen bekannt, für die eine hautaufhellende Wirksamkeit beschrieben wird. U.a. zu nennen sind hier Hexadecen-1,16-dicarbonsäure, Kojic-Säure und Derivate, Arbutin, Ascorbinsäure und Derivate, Flavonoide, Ellagsäure und Derivate, Tranexamsäure und verschiedene Resorcinol-Derivate, wie z.B. 4-n-Butylresorcin, 4-n-Hexylresorcin und 4-(1-phenylethyl)benzen-1,3-diol.

J.M. Ready beschreibt in einer Publikation (Bioorganic & Medicinal Chemistry Letter 17 (2007) 6871-6875 die Wirkung von u.a. substituierten Thiazol-Derivaten zur Inhibition der Mushroom tyrosinase.

In der Patentanmeldung der Firma Shiseido (WO 2009099195) werden substituierte Thiazolamine bzw. Hydrothiazolamine zur Hautaufhellung beschrieben.

Die im oben genannten Stand der Technik beschriebenen Substanzen weisen sich durch eine moderate Wirksamkeit und/oder eine schlechte galenische Stabilität aus.

Augenringe können ebenfalls als Folgen einer Pigmentierungsstörung entstehen, wobei sie ferner auch als Reaktion auf allgemeinen Stress, wie z.B. wenig Schlaf oder schlicht durch Überanstrengung der Augen erscheinen. Bei jüngeren Menschen verschwinden die Symptome nach ausreichender Nachtruhe wieder, über längere Zeiträume jedoch kann der Zustand chronisch und für die betroffenen Personen sehr störend werden. Auch gegen solche Hauterscheinungen mangelt es an genügend erfolgversprechenden Wirkstoffen und Behandlungsmöglichkeiten.

Ziel der nachfolgenden Erfindung war es also, dem nachteiligen Stand der Technik Abhilfe zu verschaffen.

Die Lösung der Aufgaben, die der Erfindung zugrunde liegen, besteht in Heteroalkylamidothiazole, dadurch gekennzeichnet, dass sie eine der folgenden Strukturen aufweisen: 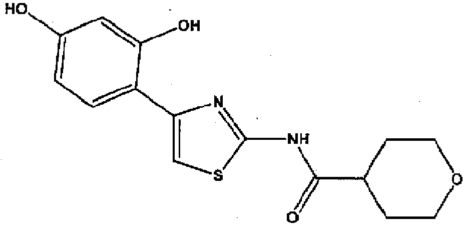 *N*-(4-(2,4-dihydroxyphenyl)thiazol-2-yl)tetrahydro-2*H*-pyran-4-carboxamide 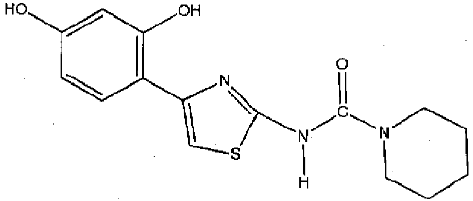 *N*-(4-(2,4-dihydroxyphenyl)thiazol-2-yl)piperidine-1-carboxamide 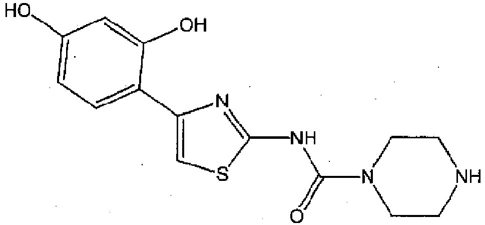 *N*-(4-(2,4-dihydroxyphenyl)thiazol-2-yl)piperazine-1-carboxamide
und 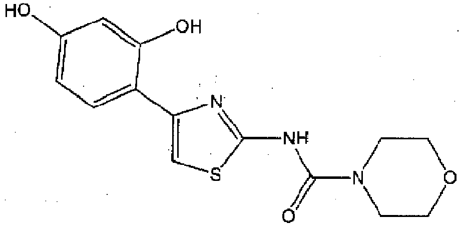 *N*-(4-(2,4-dihydroxyphenyl)thiazol-2-yl)morpholine-4-carboxamide

Die genannten Thiazole können sowohl als freie Base wie auch als Salz vorliegen: z.B. als Fluorid, Chlorid, Bromid, Iodid, Sulfat, Carbonat, Ascorbat, Acetat oder Phosphat. Im Besonderen als Halogensalze, wie z.B. Chlorid und Bromid.

Weiterhin besteht eine vorteilhafte Verwirklichung der vorliegenden Erfindung in kosmetischen oder dermatologischen Zubereitungen mit einem wirksamen Gehalt an einem oder mehreren vorbekannten Heteroalkylamidothiazolen.

Erfindungsgemäß ist ferner die Verwendung der vorgenannten Heteroalkylamidothiazole zur Behandlung und/oder Prophylaxe unerwünschter Hautpigmentierung.

Dabei können Behandlung und/oder Prophylaxe unerwünschter Hautpigmentierung sowohl im kosmetischen wie im pharmazeutischen Rahmen erfolgen.

Dabei, wird die pharmazeutische (oder dermatologische) Behandlung in erster Linie bei krankhaften Hautzuständen verstanden, wogegen die kosmetische Behandlung und/oder Prophylaxe unerwünschter Hautpigmentierung in erster Linie die gesunde Haut betrifft.

Überraschenderweise konnte gezeigt werden, daß die erfindungsgemäßen Heteroalkylamidothiazole im Vergleich zu den entsprechenden Heteroalkylaminothiazole eine höhere galenische Stabilität aufweisen und/oder eine gesteigerte Wirksamkeit. Siehe Tabelle 1.

### Methodenbeschreibung der Wirksamkeitsuntersuchungen:

Die Wirksamkeit der Thiazole wurde mit einem Enzymtest belegt, in der Umsatz von L-DOPA zu L-Dopachinon durch eine humane Tyrosinase gemessen wurde. Bei dieser literaturbekannten Methode (Winder, A.J. and Harris, H., New assays for the tyrosine hydroxylase and dopa oxidase activities of tyrosinase. Eur. J. Blochem. (1991), 198, 317-26) wird das Reaktionsprodukt L-Dopaquinone mit MBTH (3-methyl-2-benzothiazoline hydrazone) zu einer pinkfarbenen Substanz umgesetzt, deren Zunahme über die Zeit durch Absorption bei 490 nm gemessen wird. In Tabelle ein sind Wirksamkeitsdaten für einige der beanspruchten Substanzen beispielhaft dargestellt. Daraus lässt sich schließen, dass die erfindungsgemäßen Substanzen äußerst effektive pigmentierungs-inhibierende Substanzen sind.

| **Tabelle 1:** | | | |
|---|---|---|---|
| Substanz | *Inhibition (% der Kontrolle*) | *Konzentration* | |
| N-(4-(2,4-dihydroxyphenyl)thiazol-2-yl)piperidin-1-carboxamid | 97 | 10 µg/mL | Amid |
| 4-(2-(Piperidin-1-yl)thiazol-4-yl)benzen-1,3-diol | 82 | 10 µg/mL | Amin |
| N-(4-(2,4-dihydroxyphenyl)thiazol-2-yl)-4-ethylpiperazin-1-carboxamid | 94 | 10 µg/mL | Amid |
| 4-(2-(4-Ethylpiperazin-1-yl)thiazol-4-yl)benzen-1,3-diol | 91 | 10 µg/mL | Amin |
| N-(4-(2,4-dihydroxyphenyl)thiazol-2-yl)piperazin-1-carboxamid | 94 | 10 µg/mL | Amid |
| 4-(2-(Piperazin-1-yl)thiazol-4-yl)benzen-1,3-diol | 93 | 10 µg/mL | Amin |
| N-(4-(2,4-dihydroxyphenyl)thiazol-2-yl)morpholin-4-carboxamid | 97 | 10 µg/mL | Amid |
| 4-(2-Morpholinothiazol-4-yl)benzen-1,3-diol | 91 | 10 µg/mL | Amin |

### Synthesevorschriften exemplarisch ausgewählter Heteroalkylamidothiazole:

### 2-Brom-2',4'- bis-methoxycarbonyloxy-acetophenon:

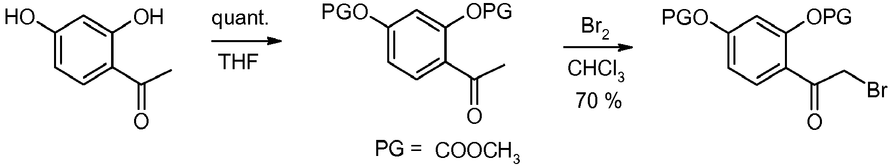

### Mitchell, David; Doecke, Christopher W.; Hay, Lynne A.; Koenig, Thomas M.; Wirth, David D. Tetrahedron Letters, 1995

Eine Lösung von 60g (369 mmol) 2,4-Dihydroxyacetophenon and 186 ml Triethylamin in 900 ml Tetrahydrofuran wurde auf 0°C gekühlt und 93 ml Chlorameisensäuremethylester in 400 ml Tetrahydrofuran langsam zugetropft. Es bildet sich ein weißer Niederschlag. Nach 3 Stunden Rühren bei Raumtemperatur ist die Reaktion abgeschlossen (DC-Kontrolle). Der Niederschlag wurde abgesaugt und mit reichlich Tetrahydrofuran gewaschen. Das Filtrat wurde zur Trockne einrotiert, in Ethylacetat aufgenommen mit 1N HCl und NaCl-Lösung (sat.) gewaschen und über Magnesiumsulfat getrocknet, vom Magnesiumsulfat filtriert und das Ethylacetat am Rotationsverdampfer eingeengt. Es wurden 105 g von 2,4-Bis-methoxycarbonyloxy-acetophenon erhalten. ¹H NMR (DMSO-D₆): 8.05 (d, 1H), 7.38 (d, 1H), 7.36 (s, 1H), 3.86 (d, 6H). Das Produkt wurde ohne weitere Reinigung eingesetzt. Zu der Lösung von 105 g 2,4-Bis-methoxycarbonyloxy-acetophenon in Chloroform (1000 ml) wurden 63g (392 mmol) Brom in 450 ml Chloroform innerhalb von 3 h zugetropft. Danach wurde die Reaktion noch 15 min. bei Raumtemperatur gerührt, Das Lösungsmittel wurde einrotiert. Der Rückstand wurde in Ethylacetat/n-Hexan verrührt, der entstandene Niederschlag wurde abgesaugt. Umkristallisation aus Ethylacetat/n-Hexan lieferten 100 g 2-Brom-2',4'- bis-methoxycarbonyloxy-acetophenon. ¹H NMR (DMSO-D₆): 8.11 (d, 1H), 7.42 (m, 2H), 4. 87 (s, 2H), 3.87 (s, 3H), 3.85 (s, 3H) ppm; m.p. 73-74°C.

### N-(4-(2,4-dihydroxyphenyl)thiazol-2-yl)tetrahydro-2H-pyran-4-carboxamid:

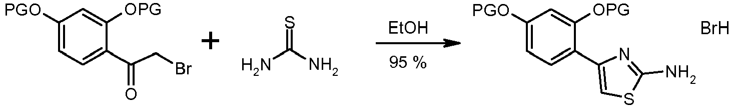

10g (28.8 mmol) 2-Brom-2',4'- bis-methoxycarbonyloxy-acetophenon wurden mit 2.20 g (28.8 mmol) Thioharnstoff in 200 ml Ethanol 1h bei 50°C gerührt. Die Reaktionslösung wurde abgekühlt, vom entstandenen Niederschlag abfiltriert, die Mutterlauge eingeengt, wobei 10.2 g eines farblosen Schaums erhalten wurden. Das so erhaltene Rohprodukt wurde ohne weitere Reinigung eingesetzt. ¹H NMR (DMSO-D₆): 7.80 (d, 1H), 7.43 (s, 1H), 7.34 (d, 1H), 5.00 (b, 3H), 3.86 (s, 3H), 3.83 (s, 3H) ppm.

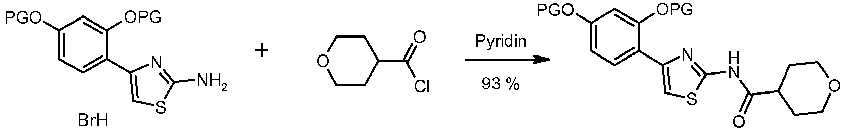

Thiazol-hydrobromid und Carbonsäurechlorid wurden in 10 ml Pyridin bei Raumtemperatur über Nacht gerührt. Das Pyridin wurde am Rotationsverdampfer abgezogen und das Reaktionsgemisch mit Wasser aufgenommen und mit Salzsäure auf pH = 1 eingestellt. Die entstandene Lösung wurde mit Ethylacetat dreimal extrahiert, die organische Phase mit Magnesiumsulfat getrocknet und einrotiert. Man erhält 2.76 g eines gelben Öls, welches ohne weitere Reinigung in die folgende Reaktion eingesetzt wurde. NMR (DMSO-D₆): 12.26 (s, 1 H), 8.00 (d, 1 H), 7.36 (m, 3H), 3.90 (m, 8H), 3.28 (m, 2H), 2.80 (m, 1 H), 1.65 (m, 4H) ppm.

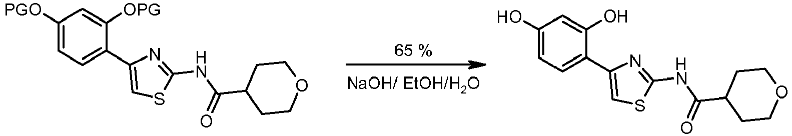

Zu der Lösung von 2.76 g Thiazol in 20 ml Ethanol wurden 0.8 g (0.019 mol) NaOH in 5 ml Wasser zugegeben und eine Stunde bei Raumtemperatur gerührt. Danach wurde die Reaktionslösung mit 30 ml Wasser aufgenommen und mit 1N HCl auf pH= 6 eingestellt. Das Ethanol wurde am Rotationsverdampfer weitgehend entfernt, der entstandene Niederschlag wurde abfiltriert und aus Ethanol/Wasser umkristallisiert. Es wurden 1.5g eines beigen Feststoffs erhalten. 500 MHz, DMSO-D₆: δ = 12.22 (bs, 1 H), 10.88 (bs, 1 H), 9.48 (bs, 1 H), 7.65 (d, 1H), 7.41 (s, 1H), 6.32 (m, 2H), 3.91 (m, 2H), 3.34 (m, 2H), 2.75 (m, 1H), 1.74 (m, 2H), 1.67 (m, 2H) ppm; m.p.: 267-272°C.

### N-(4-(2,4-dihydroxyphenyl)thiazol-2-yl)-4-ethylpiperazin-1-carboxamid:

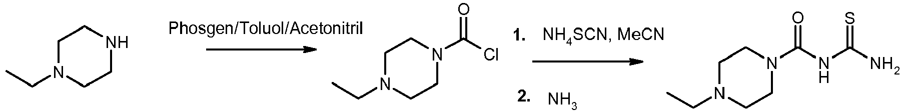

Zu der auf 0°C abgekühlten Lösung von 38 g (374 mmol) Phosgen (20 % Lösung in Toluol) in 1l Acetonitril wurde 26 g (220 mmol) Ethylpiperazin zugetropft. Dabei ist sofort ein Niederschlag ausgefallen. Das Reaktionsgemisch wurde 2 Stunden bei 60-70° C gerührt, anschließend auf 0° C abgekühlt, vom Niederschlag abfiltriert und der Niederschlag reichlich mit Acetonitril gewaschen. Das Filtrat wurde mit 3 eq NaHCO₃ behandelt. Vom erneut entstandenen Niederschlag wurde filtriert und das Lösungsmittel im Vakuum entfernt. Es wurden 19 g eines beigen Feststoffs erhalten. Das Produkt wurde ohne weitere Reinigung eingesetzt.

9 g (118 mmol) Ammoniumrhodanid wurden in 100 ml Acetonitril in der Siedehitze vorlegt und 19 g (107 mmol) Säurechlorid in 300 ml Acetonitril langsam zugetropft und danach noch 30 min unter Rückfluss gekocht. Nach dem Abkühlen auf Raumtemperatur wurde der Niederschlag abgesaugt und mit Acetonitril gewaschen. Die Mutterlauge wurde auf 0° C abgekühlt, mit 300 ml Acetonitril verdünnt und 300 ml 25 %ige wässrige Ammoniak-Lsg. rasch zugetropft. Die klare Lösung wurde 15 Min. bei 0°C, dann 1h bei Raumtemperatur gerührt und anschließend einrotiert. Die chromatographische Trennung (SiO₂, Chloroform/Methanol/NH₃ 9/1/0.1) lieferte 1.3 g farbloses Produkt. ¹H NMR (DMSO-D₆): 9.80 (s, 1 H), 9.47 (s, 1 H), 8.98 (s, 1 H), 3.41 (bs, 4H), 2.32 (m, 6H), 0.99 (t, 3H) ppm.

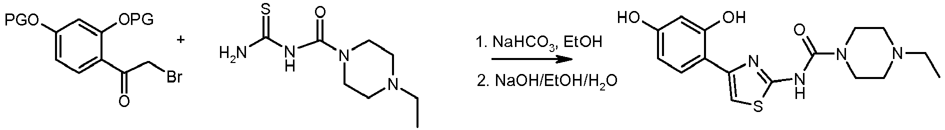

2.1 g (6.0 mmol) 2-Brom-2',4'- bis-methoxycarbonyloxy-acetophenon wurden mit 1.30 g (6.0 mmol) Thioharnstoff und 0.75 g (9.0 mmol) NaHCO₃ in 50 ml Ethanol 0.5h unter Rückfluss gekocht. Die Reaktionslösung wurde abgekühlt, mit 10 ml Wasser verdünnt und mit 1.0 g (24 mmol) NaOH in 20 ml Wasser versetzt. Nach 30 Min. Rühren bei Raumtemperatur wurde die Reaktionslösung in 30 ml Wasser aufgenommen und mit halbkonz. HCl neutralisiert und anschließend einrotiert. Die chromatographische Trennung (SiO₂, Chloroform/Methanol/NH₃ 9/1/0.1) lieferte 0.85 g Thiazol. ¹H NMR (DMSO-D₆): 11.32 (bs, 1 H), 10.94 (bs, 1 H), 9.45 (bs, 1 H), 7.58 (d, 1 H), 7.24 (s, 1 H), 6.28 (m, 2H), 3.51 (s, 4H), 2.37 (m, 6H), 1.02 (t, 3H) ppm; m.p.: 165-167°C.

### N-(4-(2,4-dihydroxyphenyl)thiazol-2-yl)piperidin-1-carboxamid:

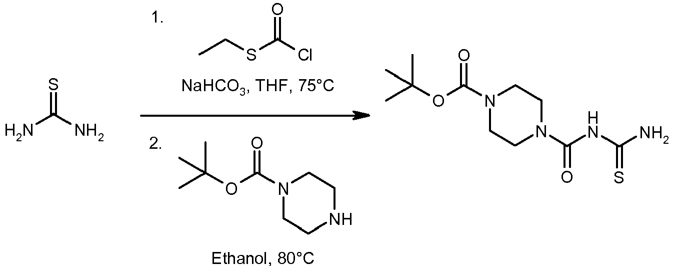

1. Schering AG, WO 2004/48343 A1
2. Brandl, Trixi; Maier Udo; Hoenke, Christoph; Joergensen, Anne T.; Pautsch, Alexander; Breitfelder, Steffen; Grauert, Matthias; Hoffmann, Matthias; Scheuerer, Stefan; Erb, Klaus; Pieper, Michael; Pragst, Ingo; US 2007/238746 A1

1.74 g (23 mmol) Thioharnstoff wurden in 40 ml THF vorgelegt, 1.3 g (17 mmol) NaHCO₃ und 1.4 g (11 mmol) Ethylchlorthioformiat zugegeben und 1 h unter Rückfluss erhitzt. Das Lösungsmittel wurde im Vakuum entfernt und anschließend das Rohprodukt per Säulenchromatographie (Kieselgel 60, CHCl₃/Methanol 9/1) gereinigt. 870 mg eines weißen Feststoffes wurden erhalten. Dieser wurde in 30 ml Ethanol aufgenommen und 1.1 g (5.8 mmol) *tert*-Butyloxycarbonylpiperazin zugegeben. Das Gemisch wurde 16 h unter Rückfluss erhitzt und anschließend auf 0°C gekühlt. Der entstandene Niederschlag wurde abgesaugt und im Vakuum getrocknet. Es resultierten 1.2 g an farblosem Feststoff. Ausbeute: 77%, ¹H NMR (DMSO-D₆): 9.89 (bs, 1 H), 9.43 (bs, 1 H), 9.02 (bs, 1 H), 3.40 (m, 4H), 3.34 (m, 4H), 1.41 (s, 9H) ppm;

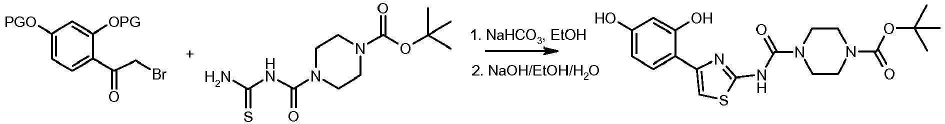

1.4 g (4.1 mmol) 2-Brom-2',4'- bis-methoxycarbonyloxy-acetophenon wurden mit 1.2 g (4.1 mmol) (*tert*-Butyloxycarbonylpiperazincarbonyl)thioharnstoff und 0.5 g (6.1 mmol) NaHCO₃ in 30 ml Ethanol unter Rückfluss für 0.5h gekocht. Die Reaktionslösung wurde abgekühlt und mit 1.0 g (25 mmol) NaOH in 7 ml Wasser versetzt. Nach 2 h Rühren bei Raumtemperatur wurde die Reaktionslösung in 50 ml Wasser aufgenommen und mit 2N HCl auf pH=7 eingestellt. Das Solvens wurde im Vakuum auf ca. 20 ml eingeengt und der Rückstand mit Essigester extrahiert. Die organische Phase wurde mit Magnesiumsulfat getrocknet, filtriert und das Solvens im Vakuum entfernt. Die Reinigung erfolgte durch Säulenchromatographie (Kieselgel, Cyclohexan/Essigester 1/1). Es wurden 1.4 g Boc-geschütztes Thiazol erhalten. Ausbeute: 82 %. ¹H NMR (DMSO-D₆): 11.27 (bs, 1 H), 11.01 (bs, 1 H), 9.45 (bs, 1 H), 7.60 (d, 1 H), 7.27 (s, 1 H), 6.29 (m, 2H), 3.50 (m, 4H), 3.38 (m, 4H), 1.42 (s, 9H) ppm. m.p.: > 225°C Zers.

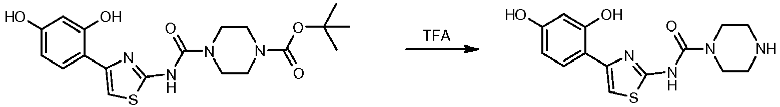

3.1 g (7.7 mmol) Boc-geschütztes Thiazol wurden in 50 ml Trifluoressigsäure aufgenommen und 16 h bei Raumtemperatur gerührt. Das Solvens wurde im Vakuum entfernt. Der erhaltene rosafarbene Feststoff wurde 20 ml in Wasser gelöst und mit 10 ml gesättigter NaHCO₃-Lösung versetzt. Der entstandene Niederschlag wurde abgesaugt und im Vakuum getrocknet. Der schwach rosafarbene Feststoff wurde dreimal jeweils in 50 ml Ethanol unter Rückfluss ausgekocht. Es resultierten 1.3 g an Thiazol. Ausbeute: 54 %. 500 MHz, ¹H NMR (DMSO-D₆): 11.37 (bs, 2H), 9.45 (bs, 1 H), 7.58 (d, 1 H), 7.22 (s, 1 H), 6.29 (m, 2H), 3.44 (m, 4H), 2.71 (m, 4H) ppm. m.p.: 320-321 °C.

### N-(4-(2,4-dihydroxyphenyl)thiazol-2-yl)morpholin-4-carboxamid:

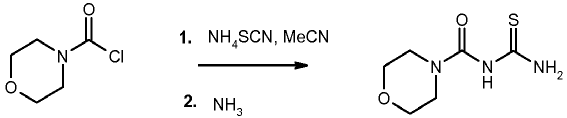

5.71 g (75 mmol) Ammoniumrhodanid wurden in 100 ml Acetonitril in der Siedehitze vorlegt und 7.48 g (50 mmol) Säurechlorid in 100 ml Acetonitril langsam zutropft und danach noch 30 min unter Rückfluss gekocht. Nach dem Abkühlen auf Raumtemperatur wurde der Niederschlag abgesaugt und mit Acetonitril gewaschen. Die gelbe Mutterlauge wurde auf 0°C abkühlt, mit 300 ml Acetonitril verdünnt und 100 ml 25 %ige wässrige Ammoniak-Lsg. rasch zugetropft. Die klare Lösung wurde 15 Min. bei 0°C, dann 1h bei Raumtemperatur gerührt und anschließend einrotiert. Die chromatographische Trennung an Kieselgel (Cyclohexan/ Ethylacetat 1/1) lieferte 1.6 g farbloses Produkt. ¹H NMR (DMSO-D₆): 9.82 (s, 1H), 9.45 (s, 1 H), 9.01 (s, 1 H), 3.56 (t, 4H), 3.41 (t, 4H) ppm.

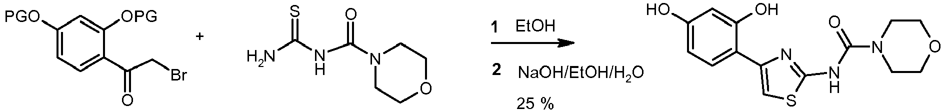

2.93 g (8.45 mmol) 2-Brom-2',4'- bis-methoxycarbonyloxy-acetophenon wurden mit 1.60 g (8.45 mmol) N-Morpholinocarbonyl-thioharnstoff und 1.07 g (12.7 mmol) NaHCO₃ in 50 ml Ethanol 0.5h unter Rückfluss gekocht. Die Reaktionslösung wurde abgekühlt, mit 10 ml Wasser verdünnt und mit 2.0 g (50 mmol) NaOH in 20 ml Wasser versetzt. Nach 30 Min. Rühren bei Raumtemperatur wurde die Reaktionslösung in 30 ml Wasser aufgenommen und mit conc. HCl neutralisiert. Der entstandene Niederschlag wurde abfiltriert und aus Ethanol/Wasser umkristallisiert. Es wurde 1.40 g Produktes erhalten. ¹H NMR (DMSO-D₆): 11.28 (bs, 1 H), 10.96 (bs, 1 H), 9.45 (bs, 1 H), 7.59 (d, 1 H), 7.27 (s, 1 H), 6.29 (m, 2H), 3.62 (m, 4H), 3.51 (m, 4H) ppm; m.p.: 268-270°C.

Kosmetische oder dermatologische Zubereitungen mit einem Gehalt an Heteroalkylamidothiazolen bzw. deren Verwendung zur Behandlung und/oder Prophylaxe unerwünschter Hautpigmentierung, sind ebenfalls vorteilhafte Verkörperungen der vorliegenden Erfindung.

Vorteilhaft ist es insbesondere, wenn solche Zubereitungen 0,000001 bis 10 Gew.-%, insbesondere 0,0001 bis 3 Gew.-%, ganz besonders 0,001 bis 1 Gew.-% an einem oder mehreren der erfindungsgemäß verwendeten Heteroalkylamidothiazolen enthalten, bezogen auf das Gesamtgewicht der Zubereitung.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindungsgemäß vorteilhaft, die erfindungsgemäß verwendeten Substanzen und/oder deren Derivate in verkapselter Form darzureichen, z.B. in Kollagenmatrices und anderen üblichen Verkapselungsmaterialien, z.B. als Celluloseverkapselungen, in Gelatine oder liposomal verkapselt.

Es ist auch möglich und vorteilhaft im Sinne der vorliegenden Erfindung, die erfindungsgemäß verwendeten Substanzen und/oder deren Derivate in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Siliconderivate.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Feuchthaltemittel wie z.B. Propylenglycol, Panthenol oder Hyaluronsäure sowie insbesondere ein oder mehrere Verdickungsmittel, welches oder welche vorteilhaft gewählt werden können aus der Gruppe Siliciumdioxid, Aluminiumsilikate, Hydroxypropylmethylcellulose, besonders vorteilhaft ein Polyacrylat wie beispielsweise Carbopole Typ 980, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Propyleglycol, und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen.

### Rezepturbeispiele

### O/W -Emulsionen

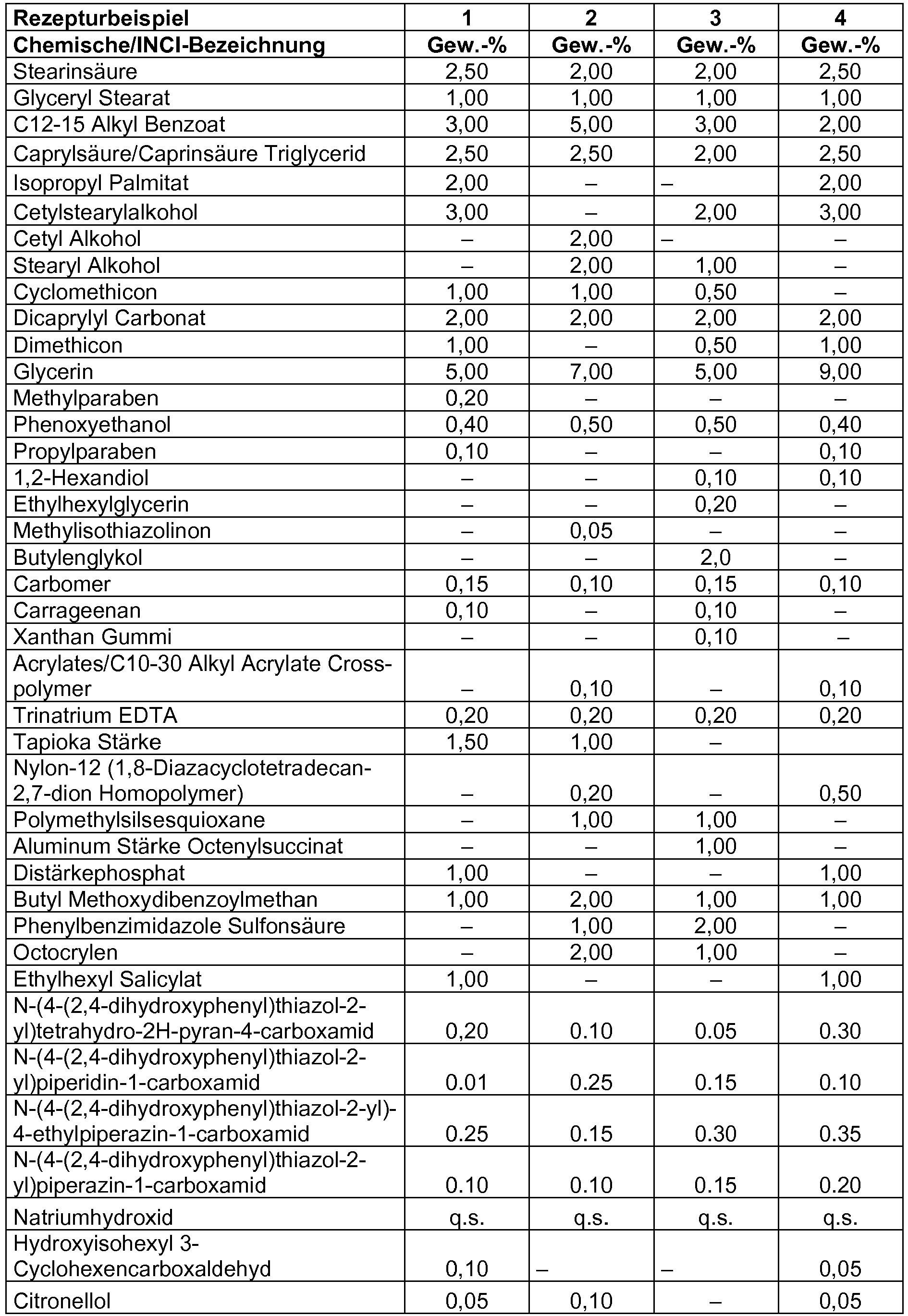

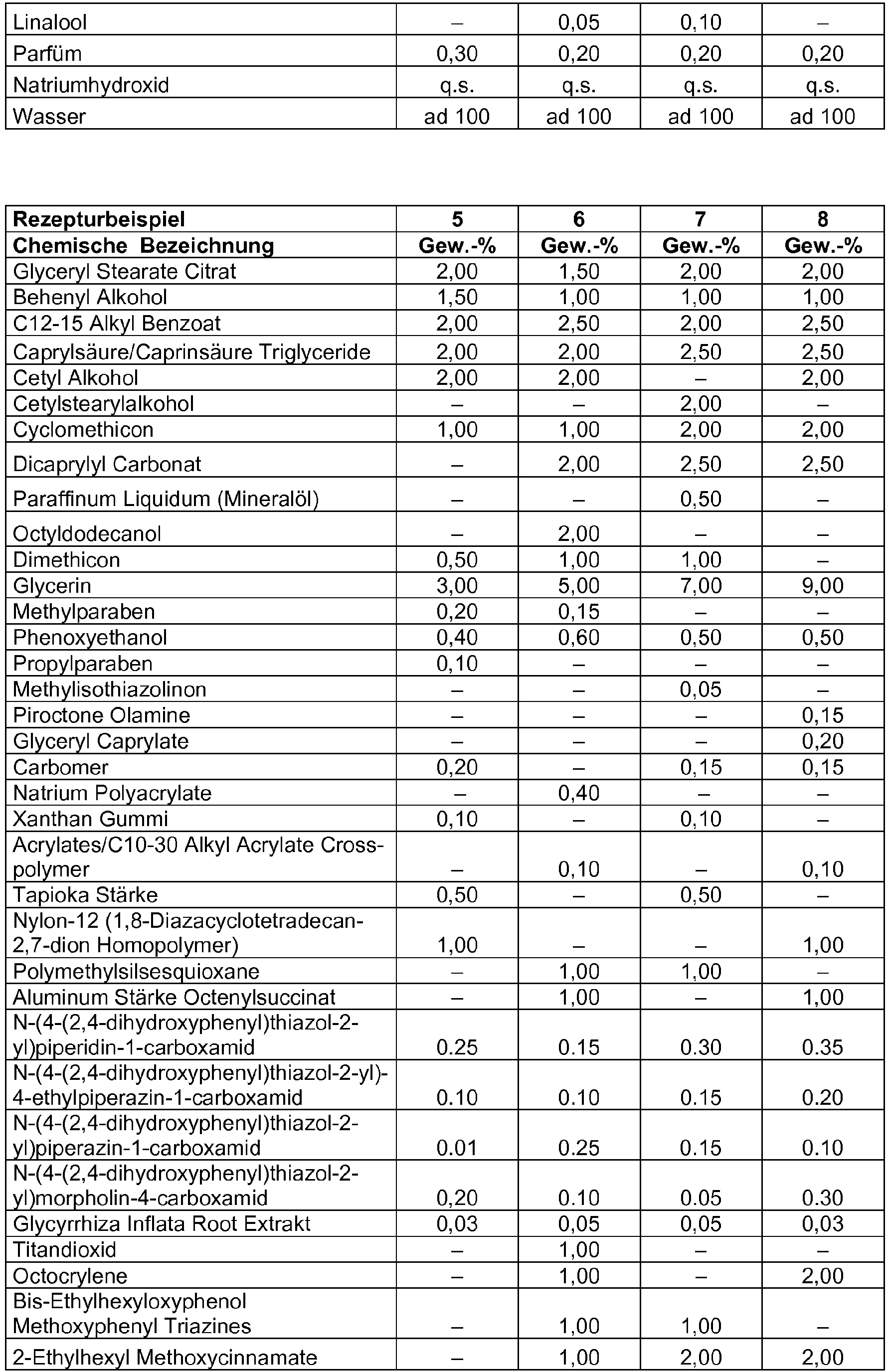

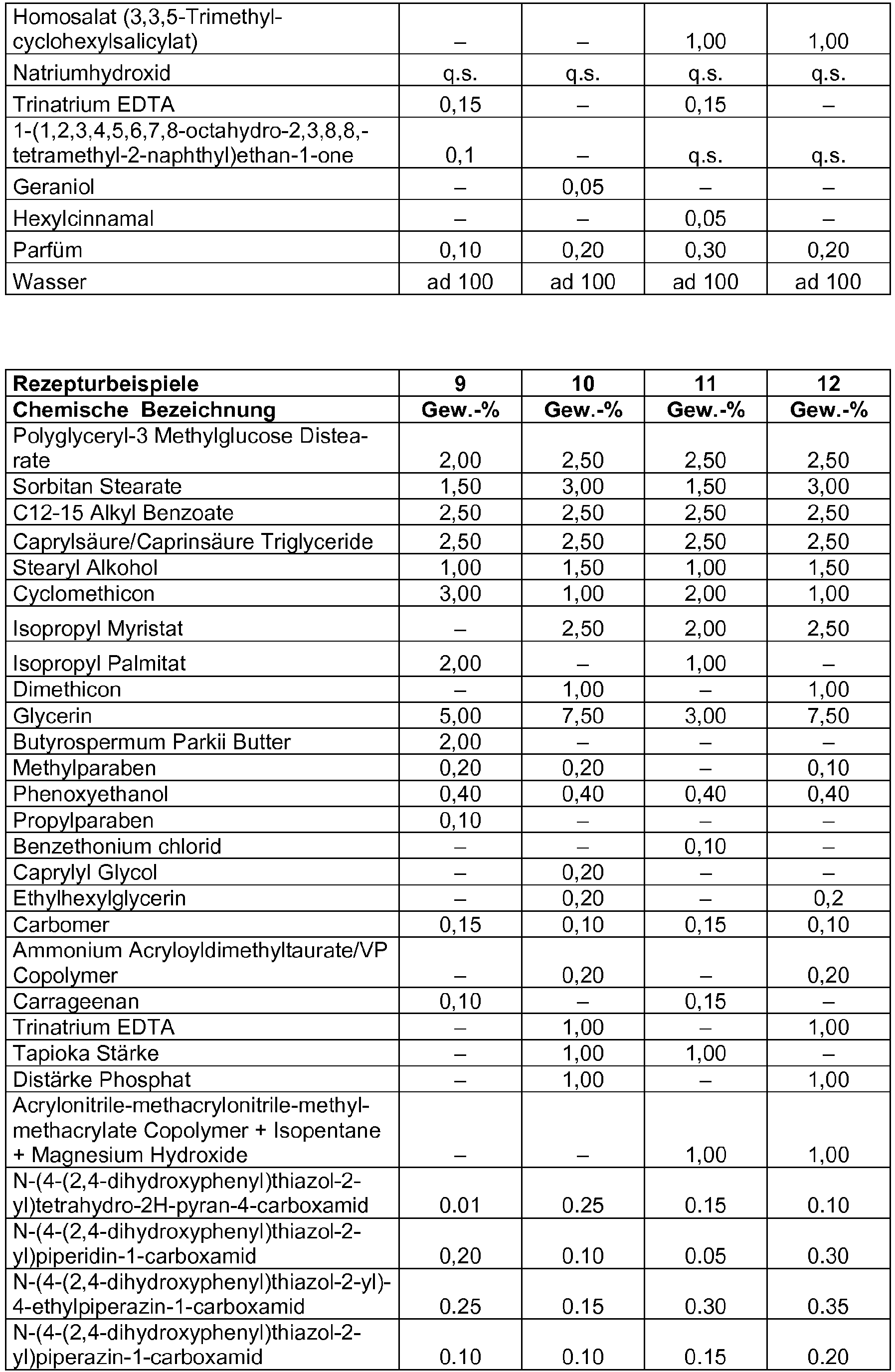

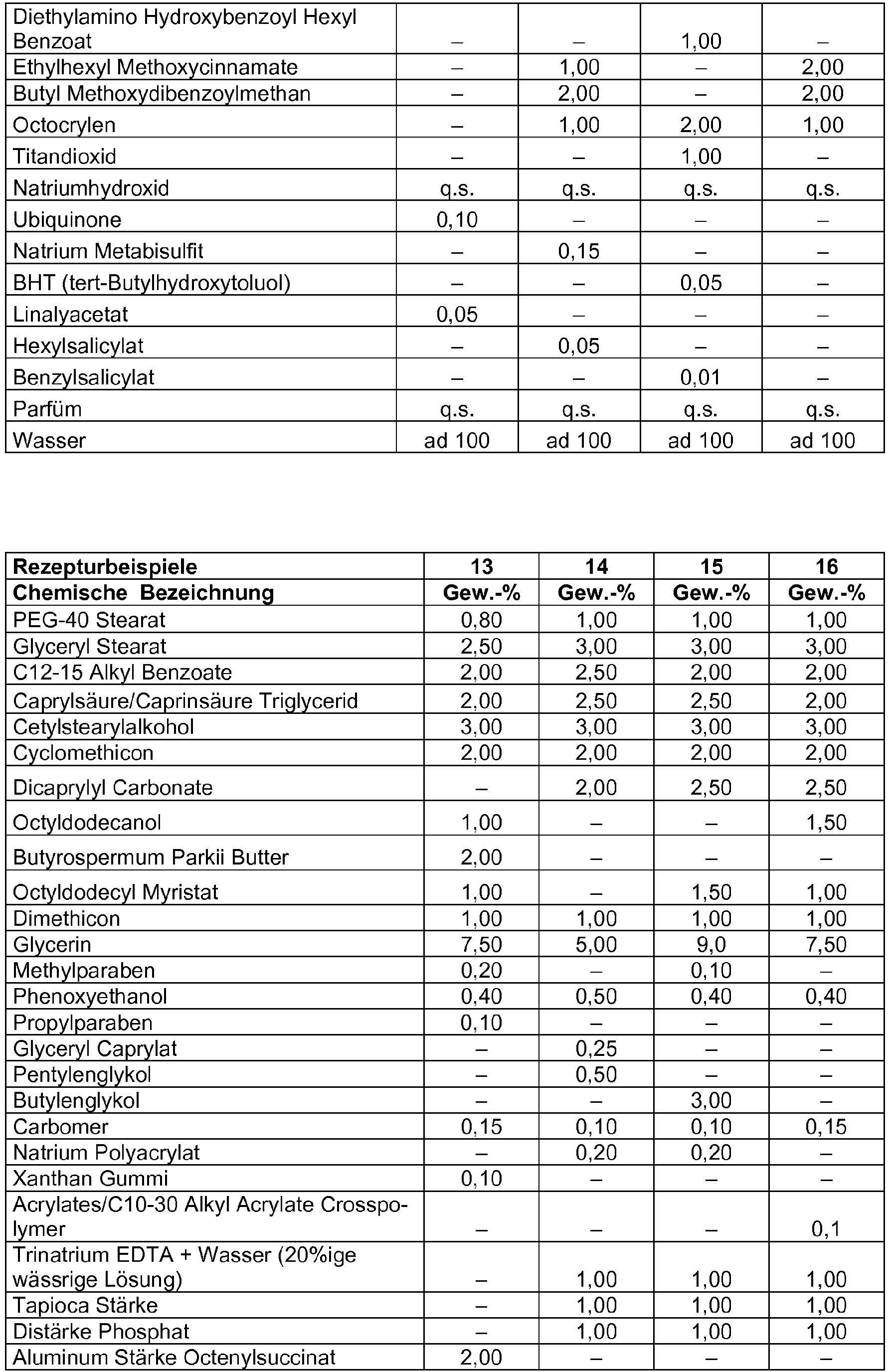

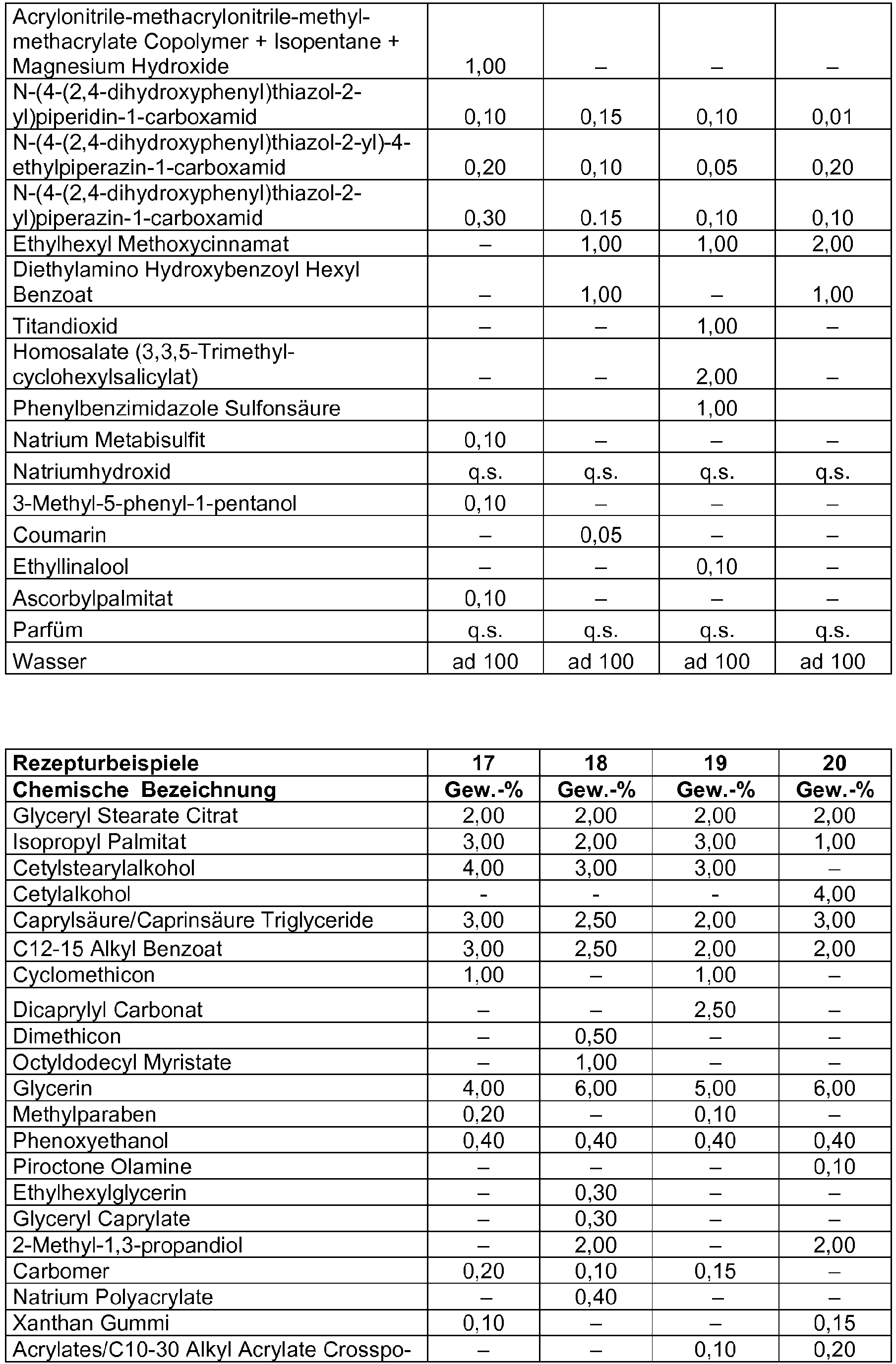

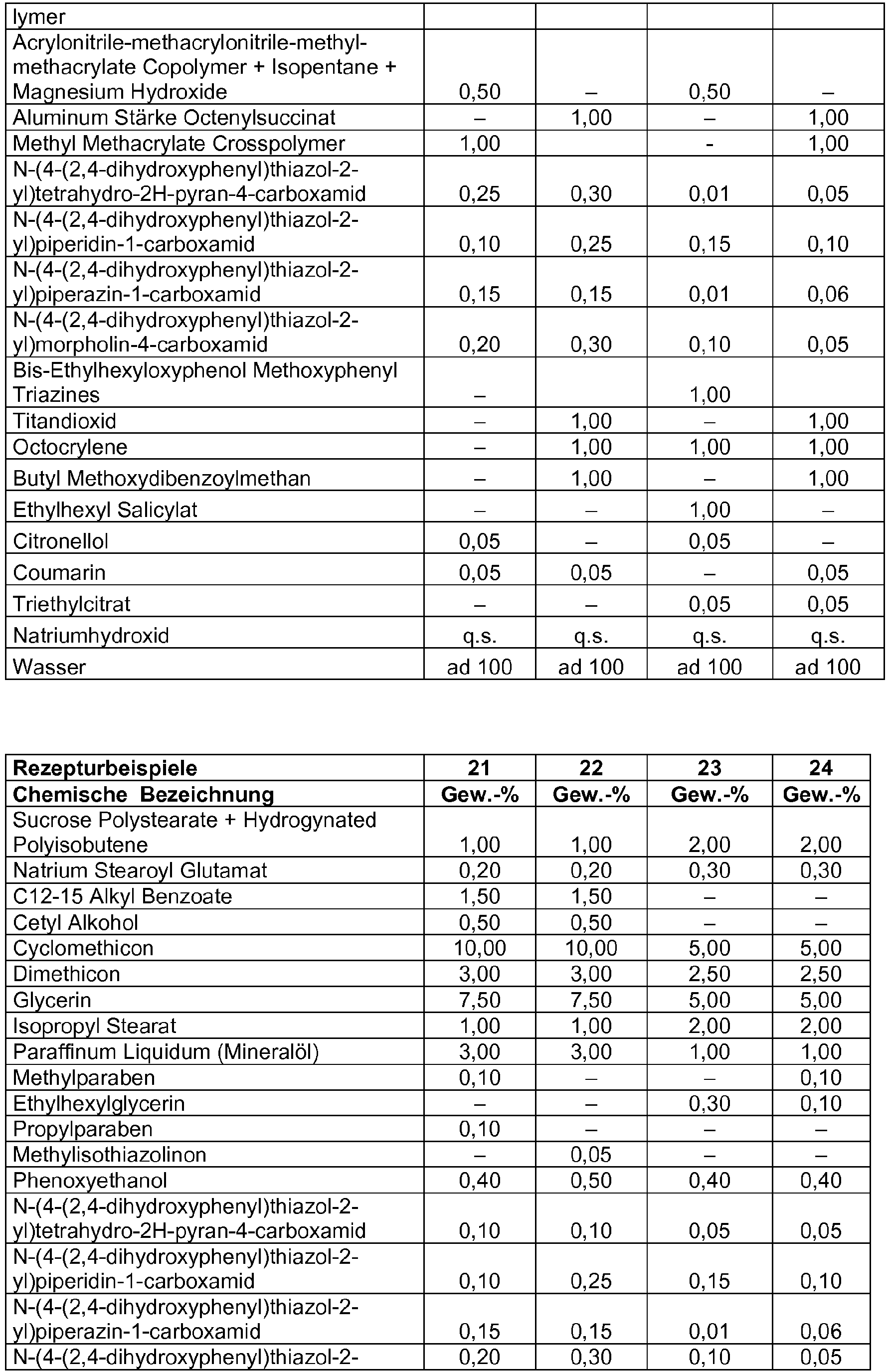

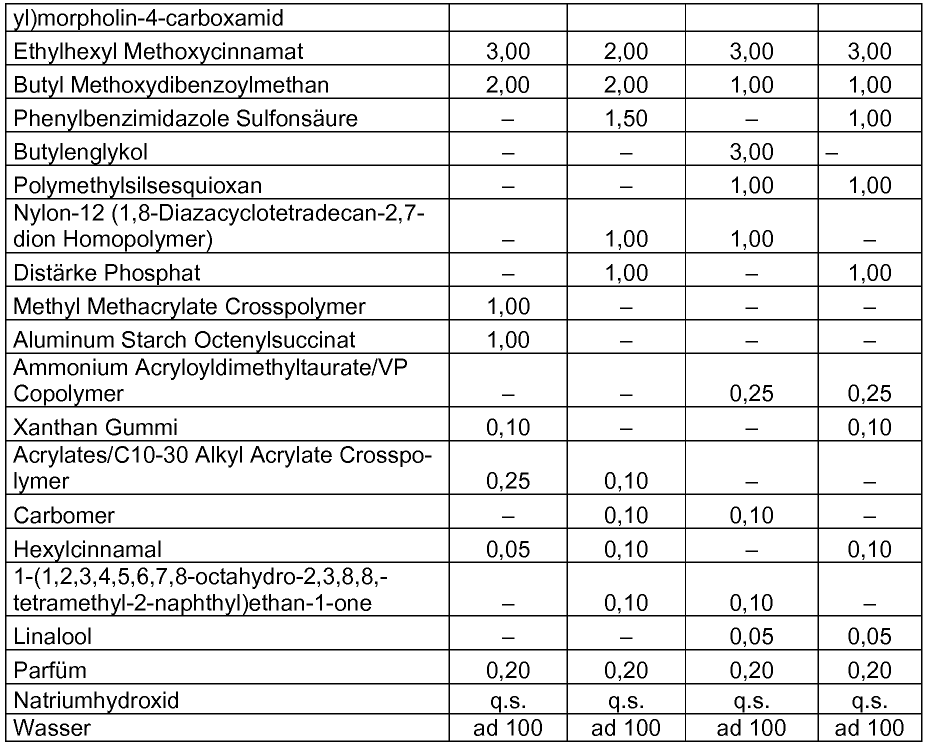

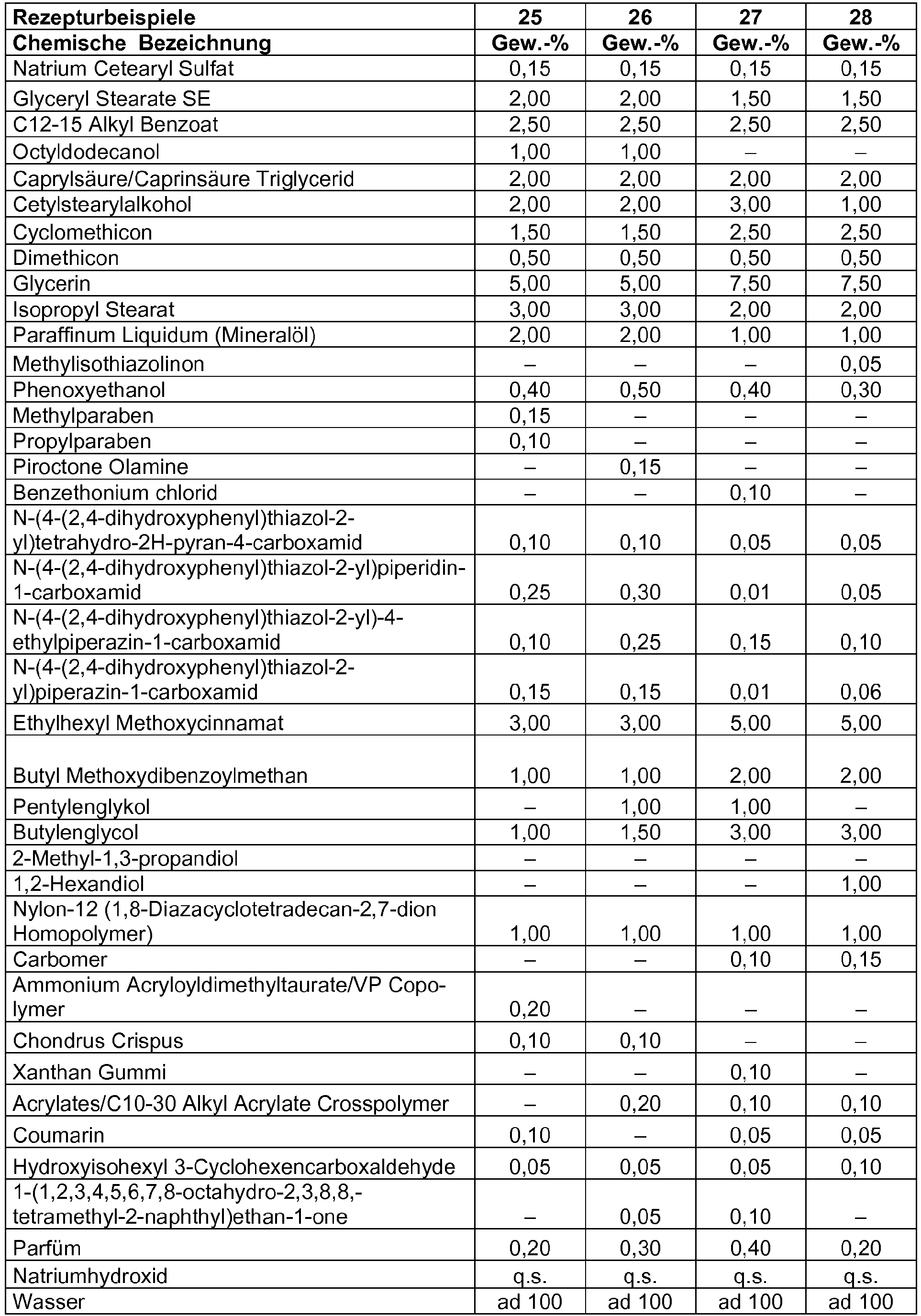

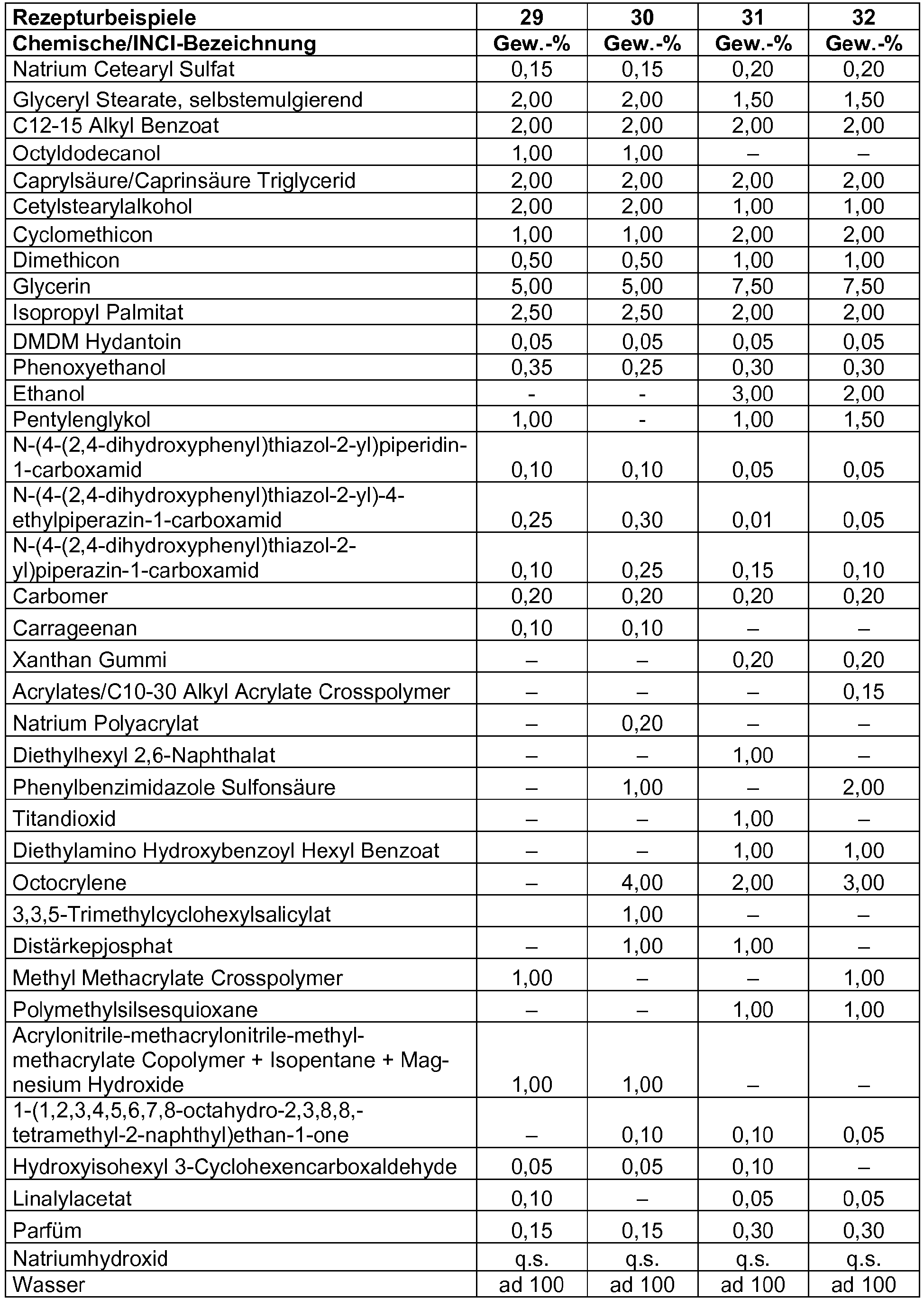

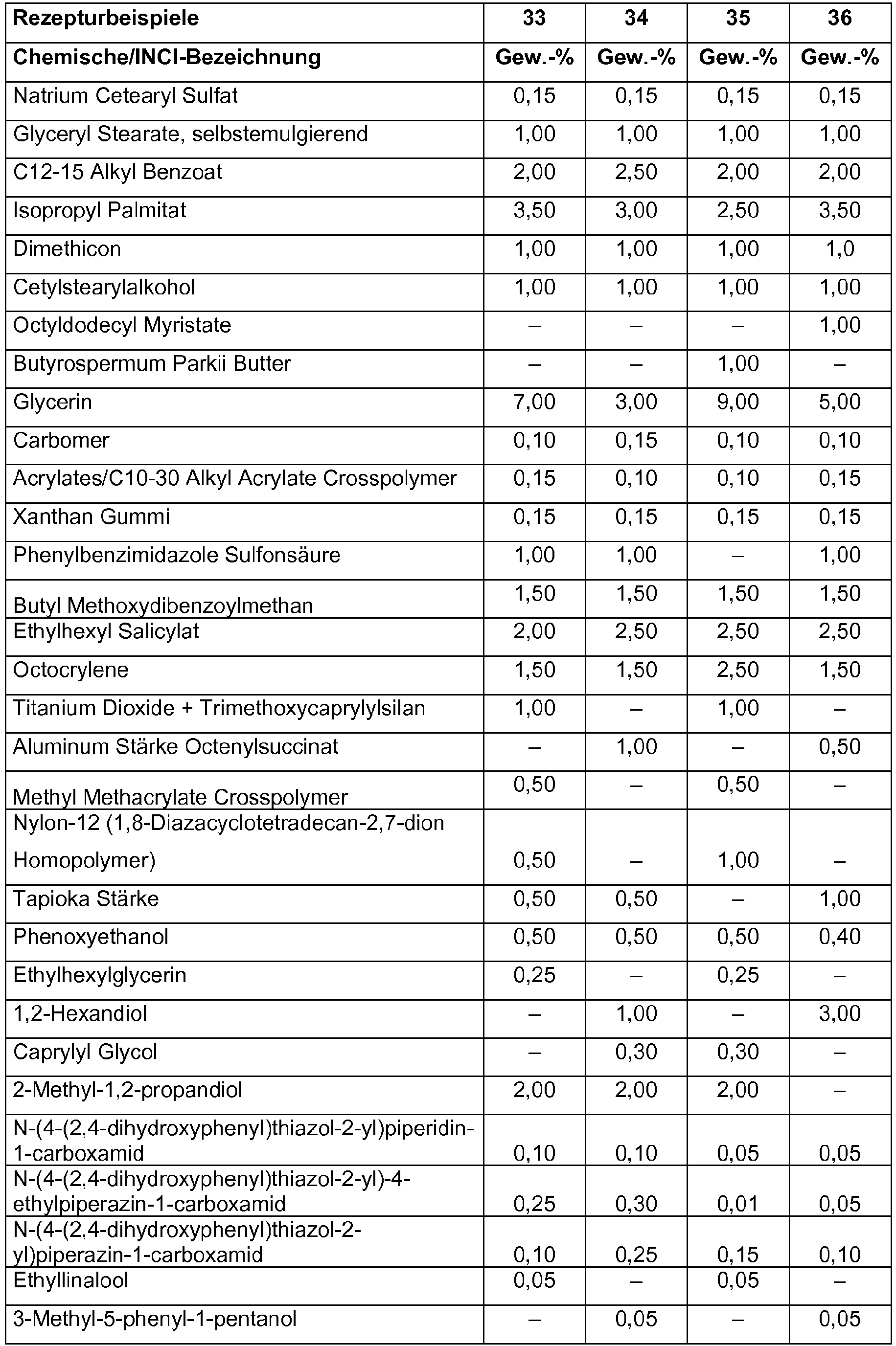

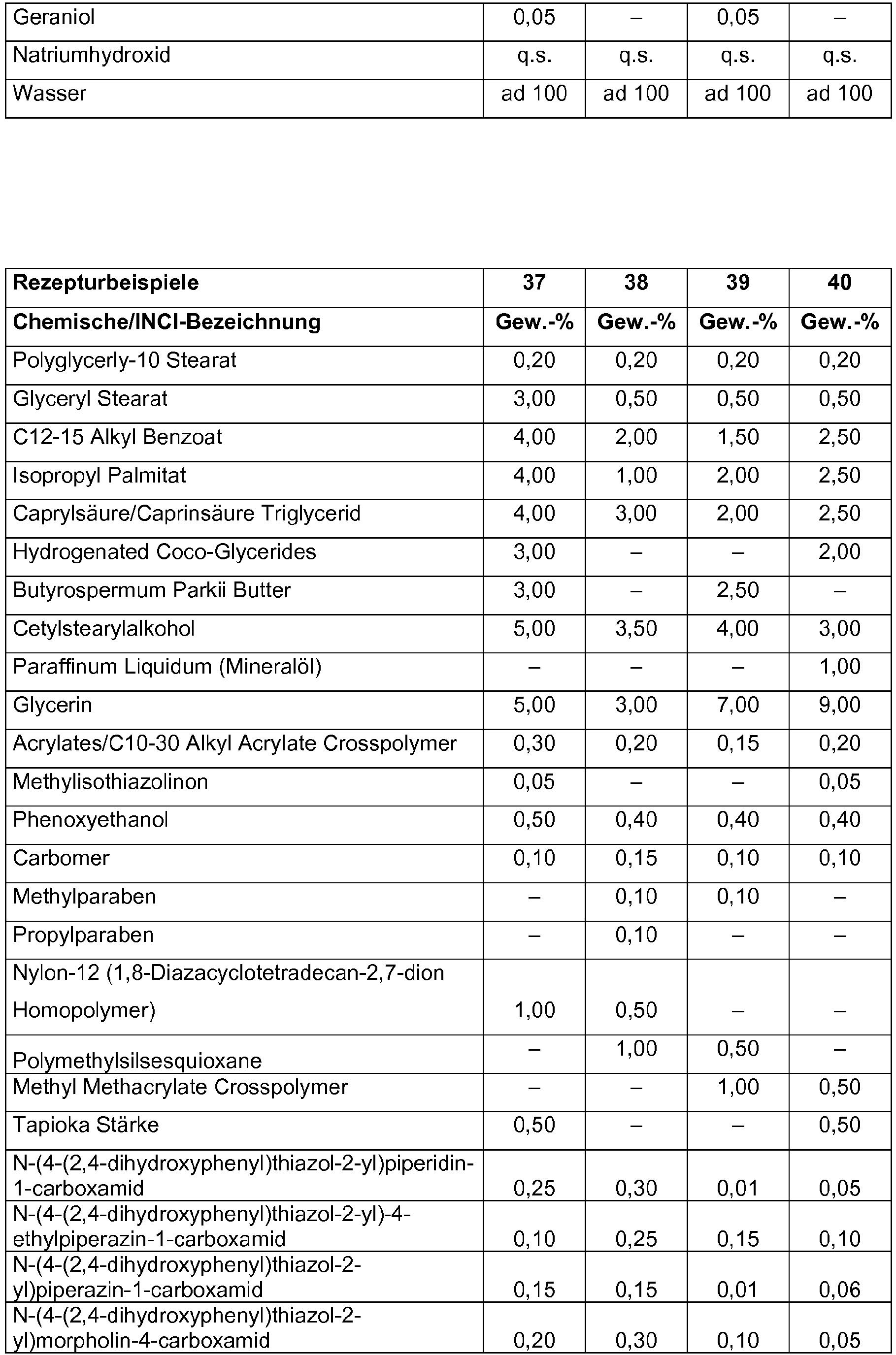

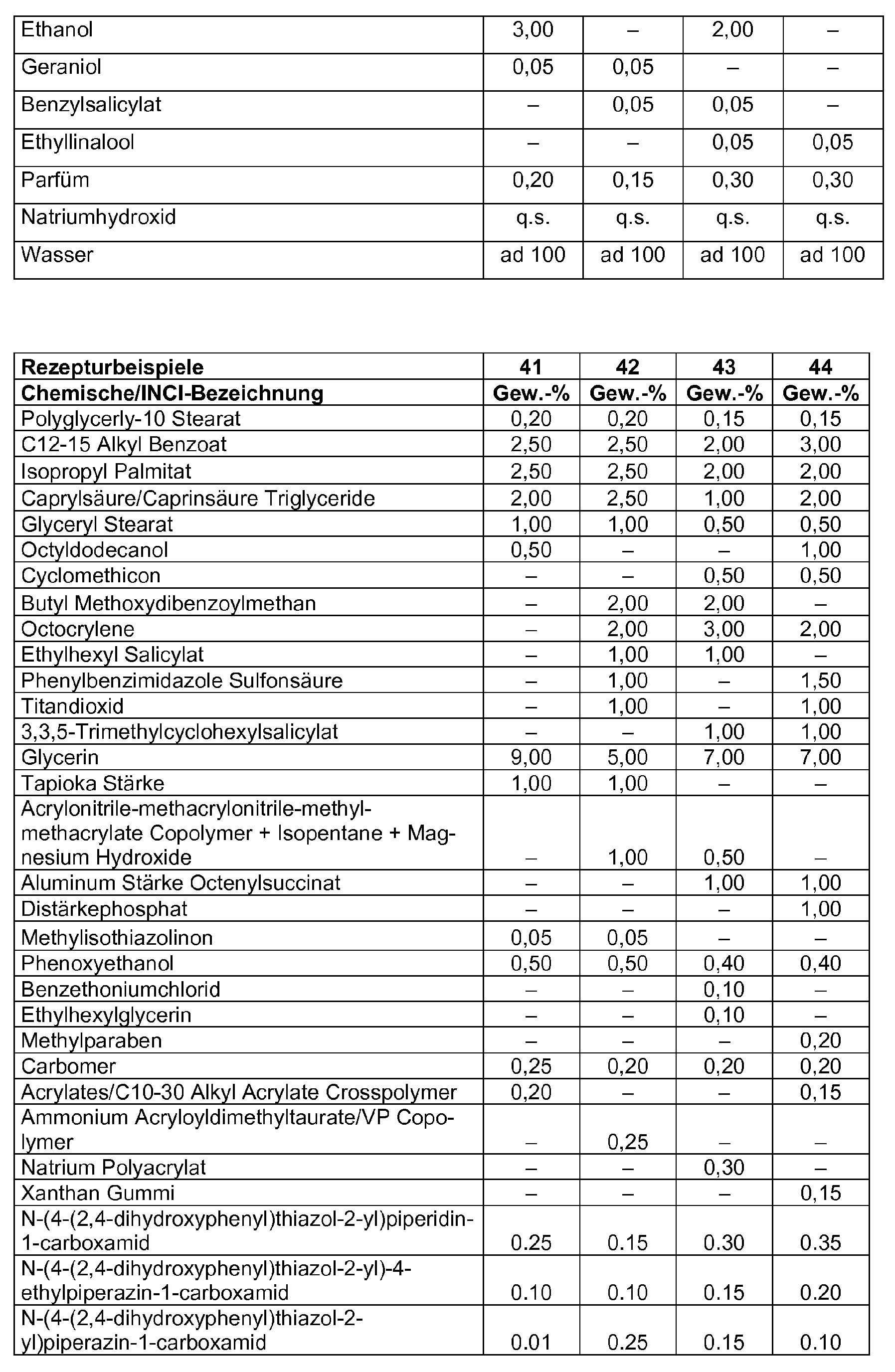

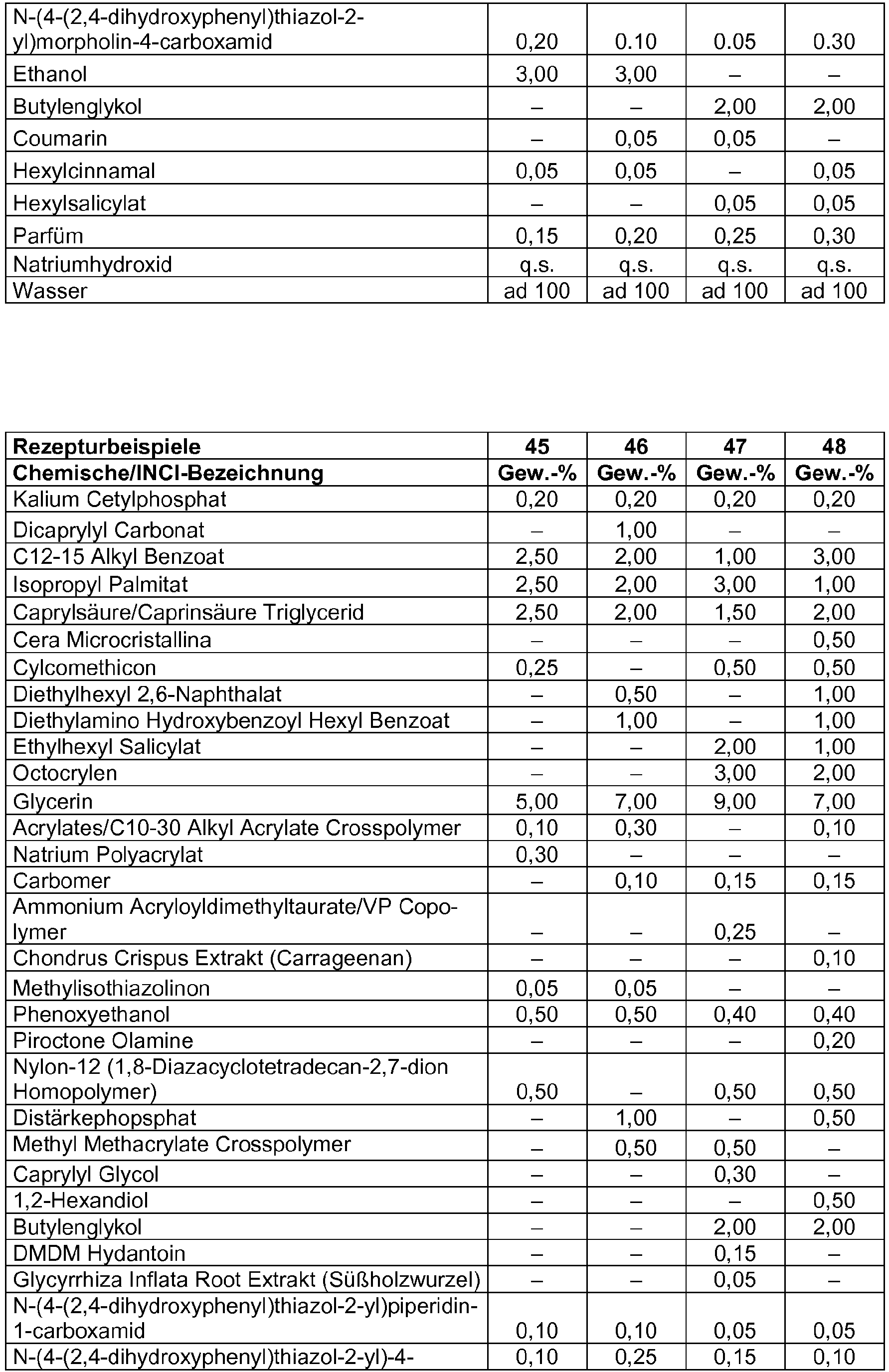

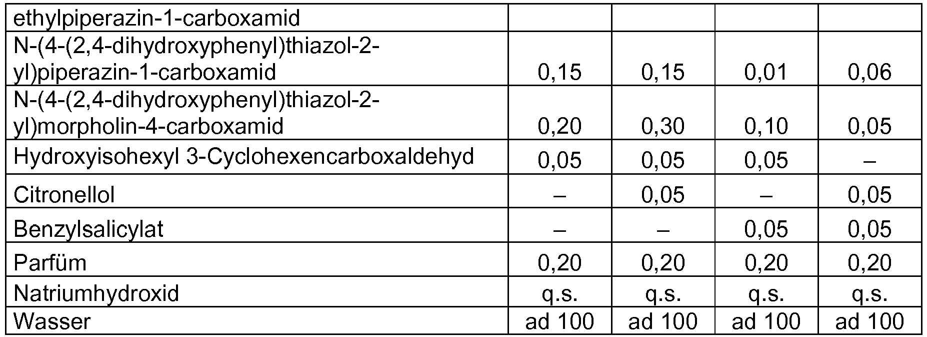

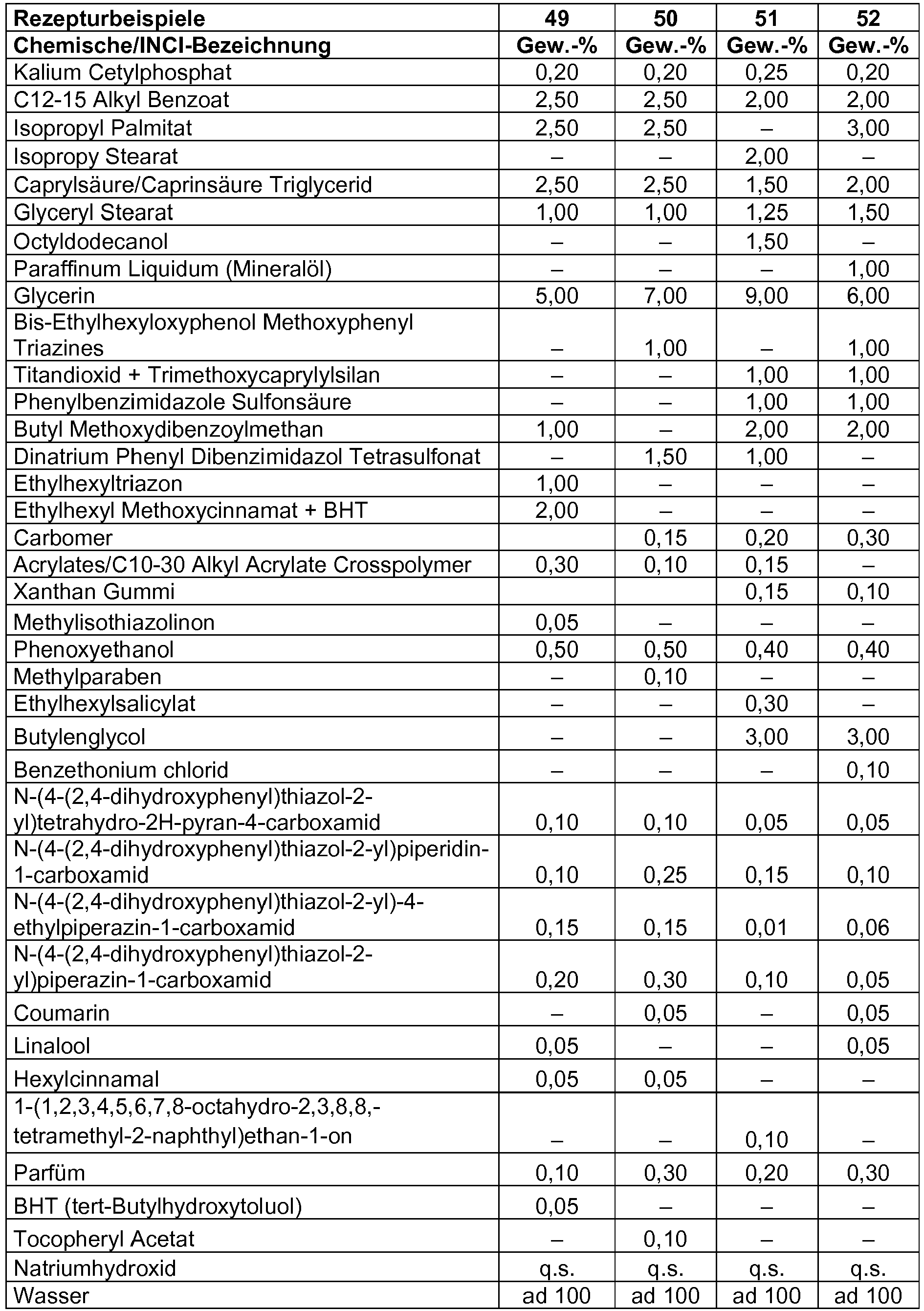

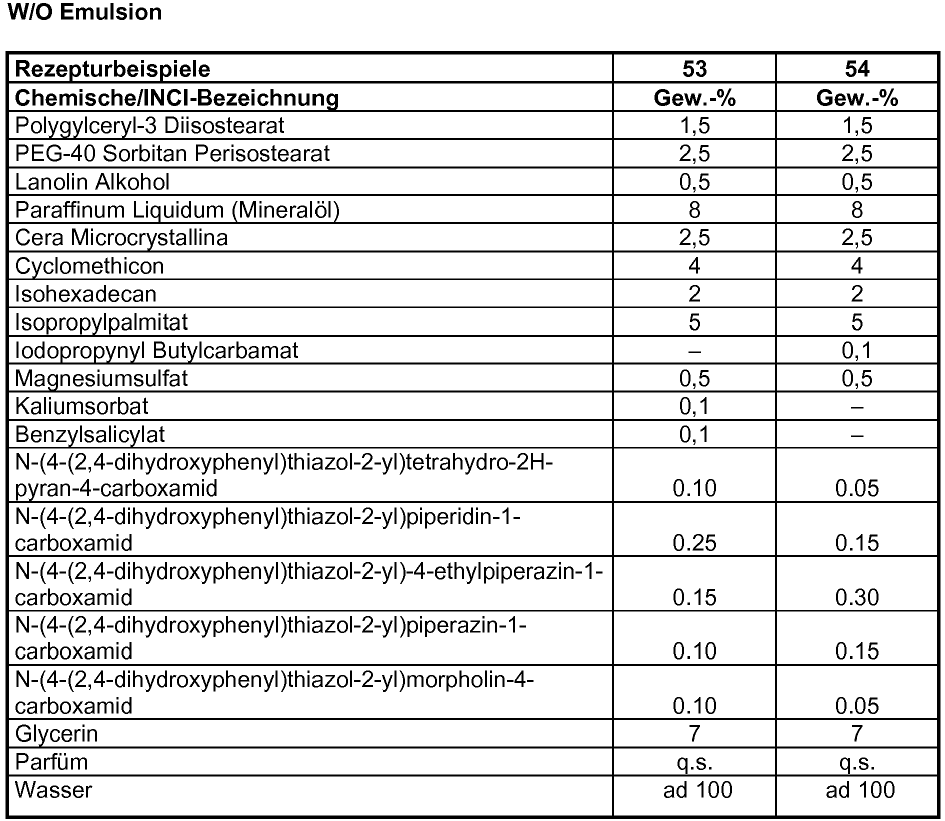

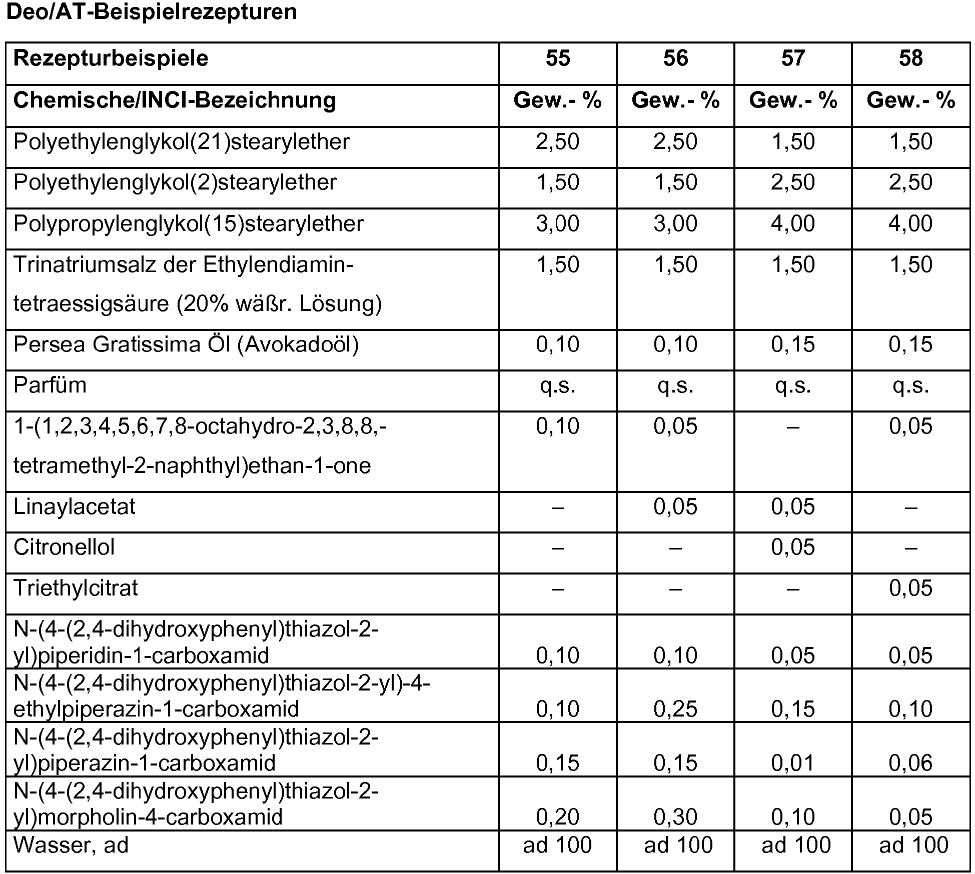

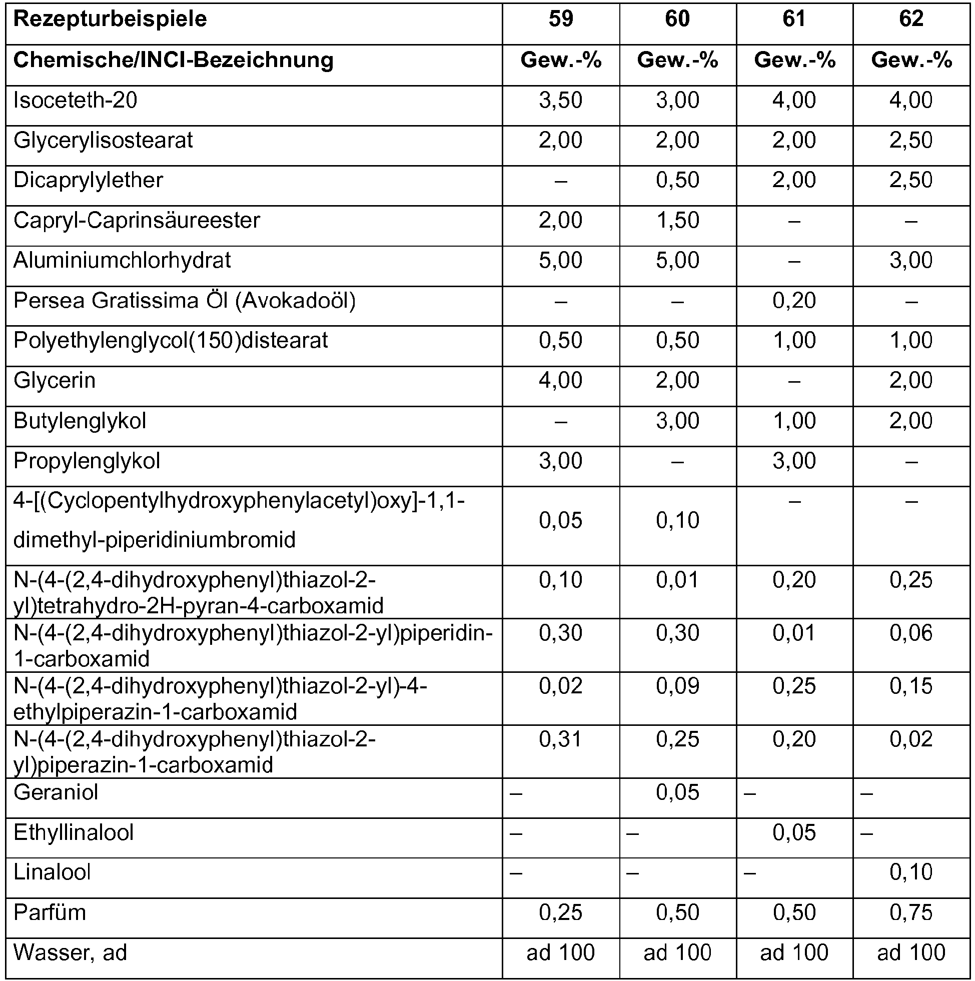

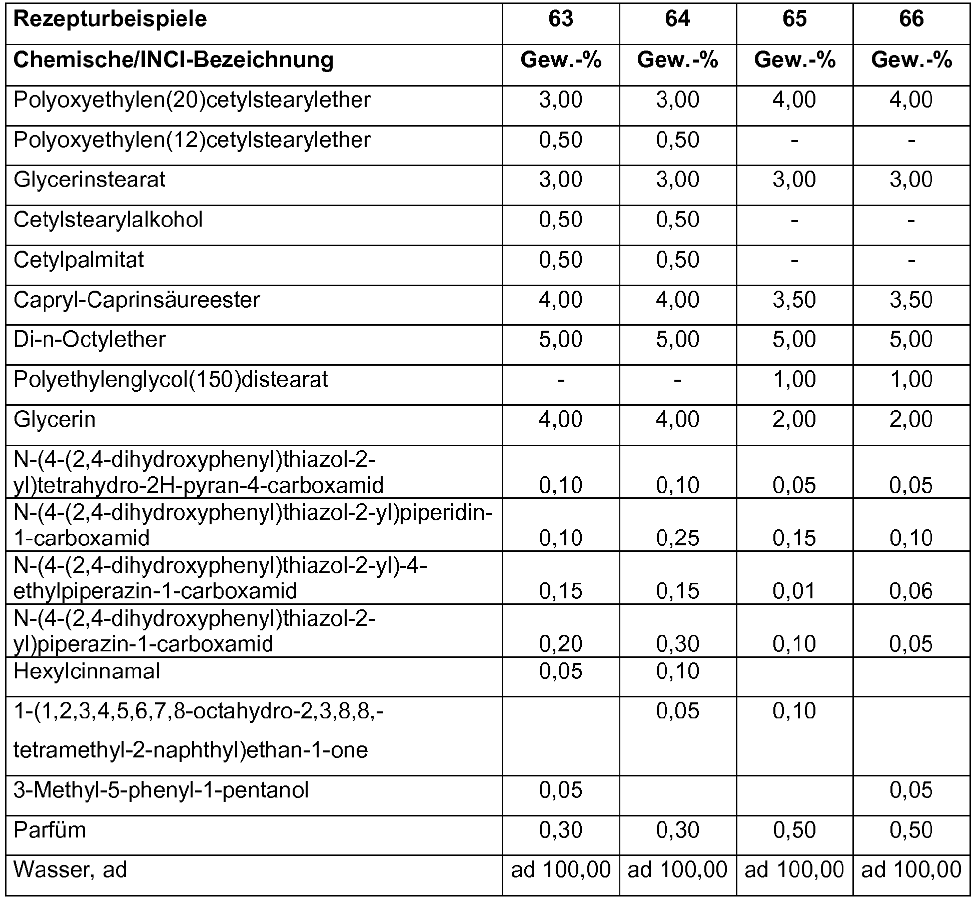

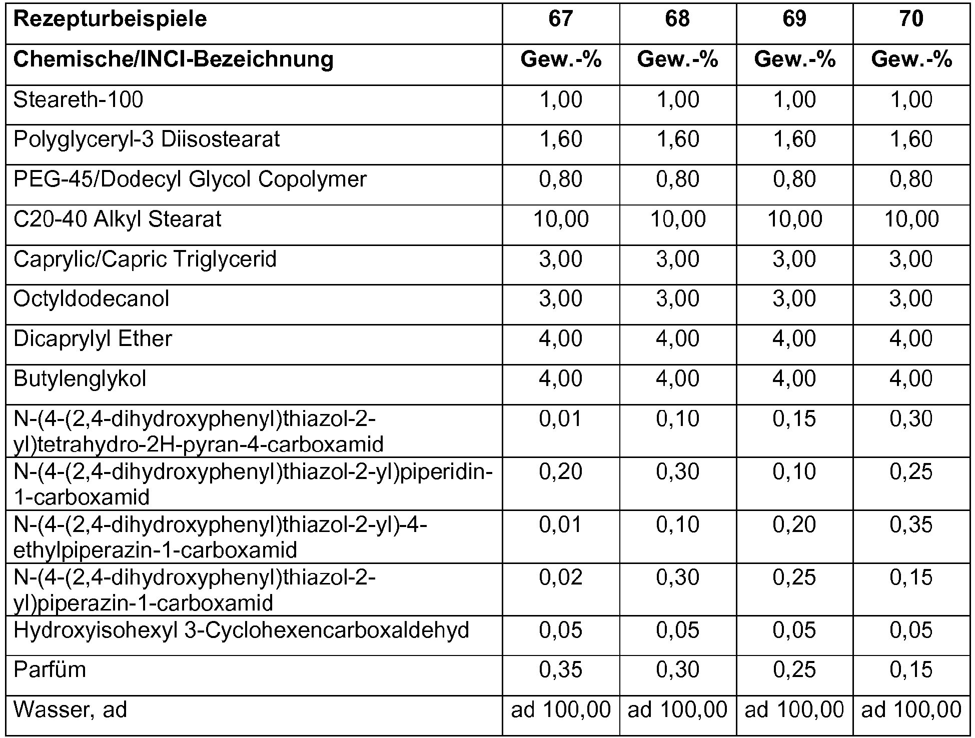

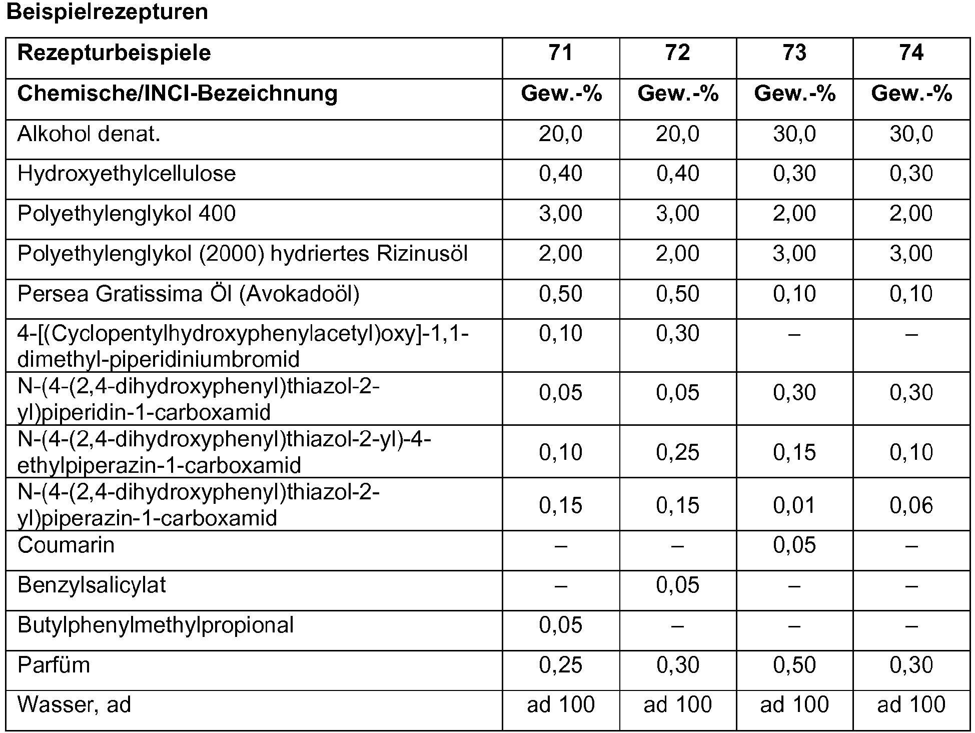

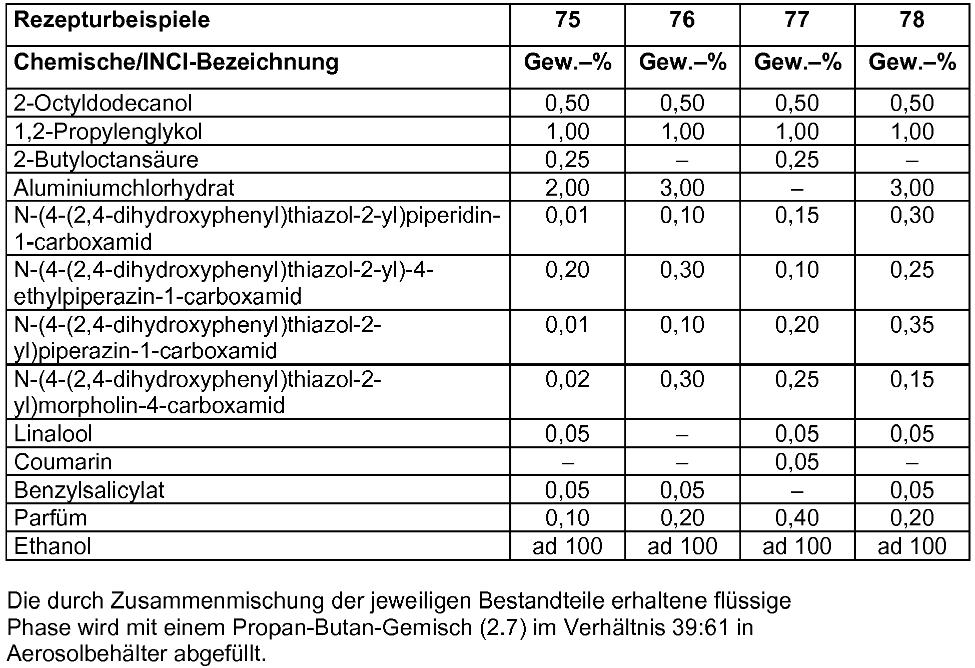

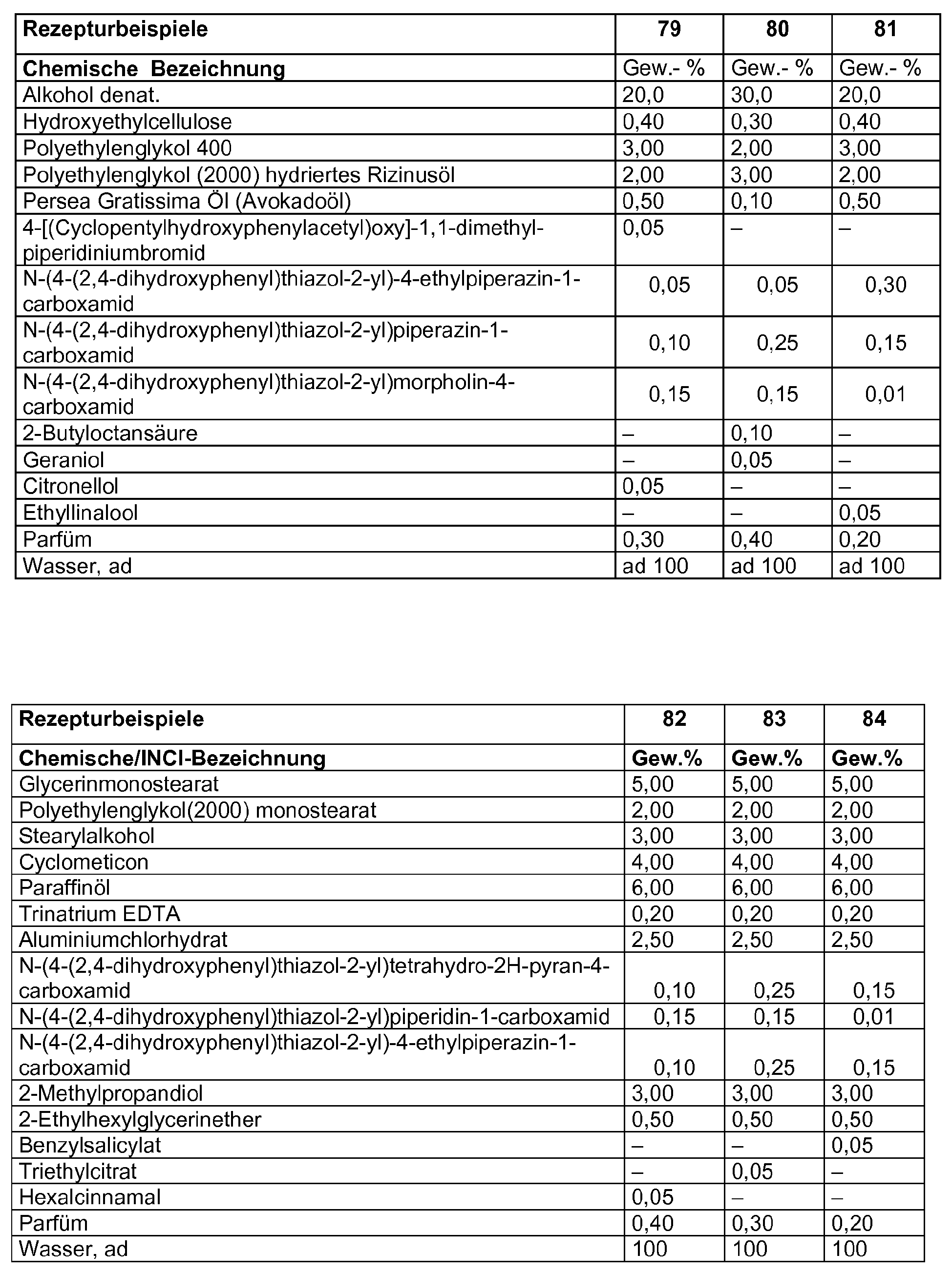

## Patentansprüche

1. Heteroalkylamidothiazole **dadurch gekennzeichnet, dass** sie eine der folgenden Strukturen aufweisen: 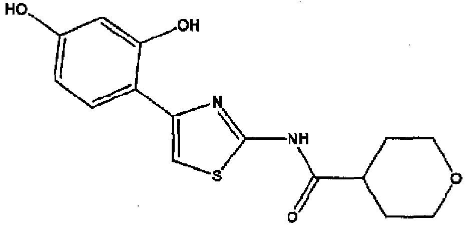 *N*-(4-(2,4-dihydroxyphetryl)thiazol-2-yl)tetrahydro-2*H*-pyran-4-carboxamide 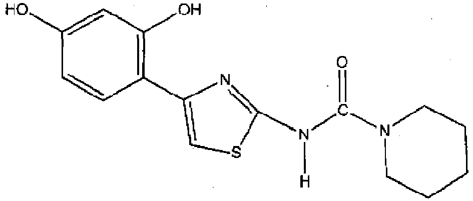 *N*-(4-(2,4-dihydroxyphenyl)thiazol-2-yl)piperidine-1-carboxamide 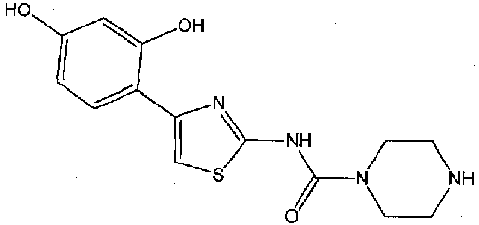 *N*-(4-(2,4-dihydrophenyl)thiazol-2-yl)piperazine-1-carboxamide und 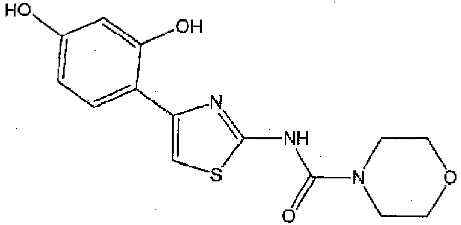 *N*-(4-(2,4-dihydroxyphenyl)thiazol-2-yl)morpholine-4-carboxamide

2. Heteroalkylamidothiazole nach Anspruch 1, **dadurch gekennzeichnet, dass** sie als Halogenid, Carbonat, Ascorbat, , Sulfat, Acetat und/oder Phosphat vorliegen.

3. Kosmetische oder dermatologische Zubereitungen mit einem Gehalt an einem oder mehreren aromatischen Heteroalkylamidothiazolen, wie sie in Anspruch 1 oder 2 definiert sind.

4. Zubereitungen nach Anspruch 3, enthaltend 0,000001 bis 10 Gew.-%, insbesondere 0,0001 bis 3 Gew.-%, ganz besonders 0,001 bis 1 Gew.-% an einem oder mehreren der in einem der Anspruch 1 oder 2 definierten aromatischen Heteroalkylamidothiazole, bezogen auf das Gesamtgewicht der Zubereitung.

5. Verwendung eines oder mehrerer in Anspruch 1 oder 2 definierten aromatischen Heteroalkylamidothiazole, oder Zubereitungen, eines oder mehrere solcher aromatischer Heteroalkylamidothiazole enthaltend, zur kosmetischen Behandlung und/oder Prophylaxe unerwünschter Hautpigmentierung.

6. In Anspruch 1 oder 2 definierte aromatischen Heteroalkylamidothiazole zur dermatologischen Behandlung und/oder Prophylaxe unerwünschter Hautpigmentierung.

## Claims

1. Heteroalkylamidothiazoles, **characterized in that** they have one of the following structures: 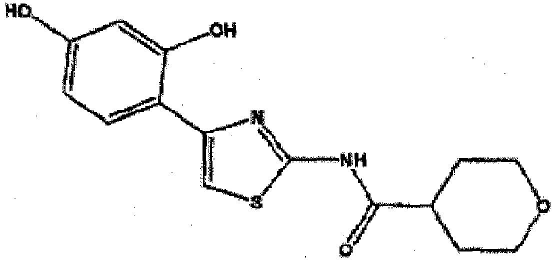 *N*-(4-(2,4-dihydroxyphenyl)thiazol-2-yl)tetrahydro-2*H-*pyran-4-carbaxamide 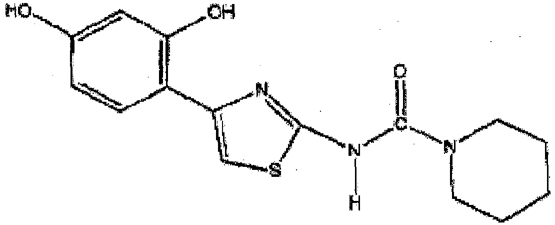 *N*-(4-(2,4-dihydroxyphenyl)thiazol-2-yl)piperidine-1-carboxamide 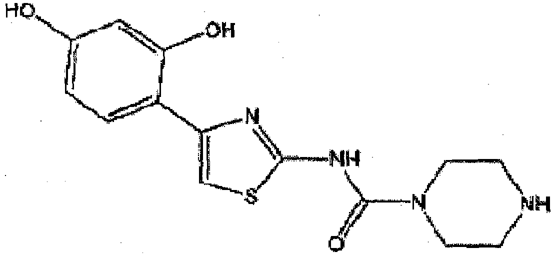 *N*-(4-(2,4-dihydroxyphenyl)thiazol-2-yl)piperazine-1-carboxamide
and 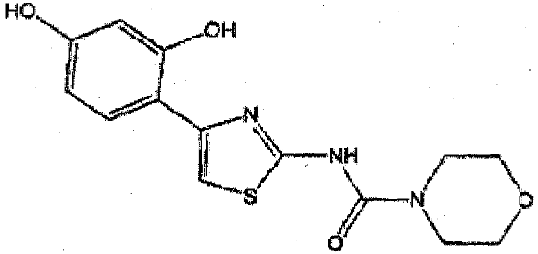 *N*-(4-(2,4-dihydroxyphenyl)thiazol-2-yl)morpholine-4-carboxamide

2. Heteroalkylamidothiazoles according to Claim 1, **characterized in that** they are present as halide, carbonate, ascorbate, sulphate, acetate and/or phosphate.

3. Cosmetic or dermatological preparations with a content of one or more aromatic heteroalkylamidothiazoles as defined in Claim 1 or 2.

4. Preparations according to Claim 3, comprising 0.000001 to 10% by weight, in particular 0.0001 to 3% by weight, very particularly 0.001 to 1% by weight, of one or more of the aromatic heteroalkylemidothiazoles defined in one of Claims 1 and 2, based on the total weight of the preparation.

5. Use of one or more aromatic heteroalkylamidothiazoles defined in Claim 1 or 2, or preparations comprising one or more of such aromatic heteroalkylamidothiazoles for the cosmetic treatment and/or prophylaxis of undesired skin pigmentation.

6. Aromatic heteroalkylamidothiazoles defined in Claim 1 or 2 for the dermatological treatment and/or prophylaxis of undesired skin pigmentation.

## Revendications

1. Hétéroalkylamidothiazoles, **caractérisés en ce qu'**ils présentent une des structures suivantes : 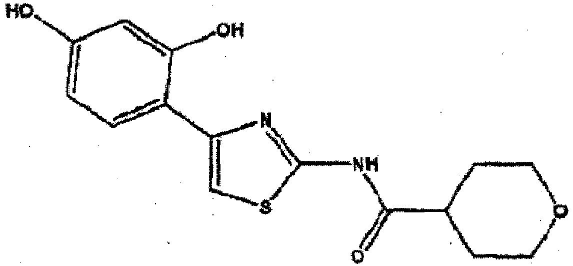 N-(4-(2,4-dihydroxyphényl)thiazol-2-yl)tétrahydro-2H-pyrane-4-carboxamide 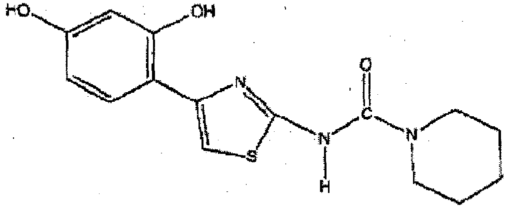 N-(4-(2,4-dihydroxyphényl)thiazol-2-yl)pipéridine-1-carboxamide 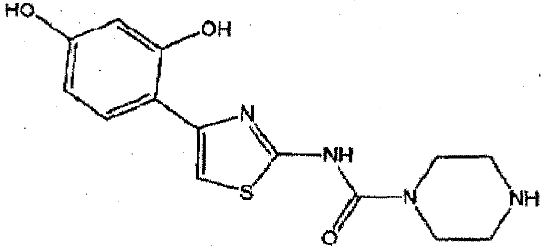 N-(4-(2,4-dihydroxyphény)thiazol-2-yl)pipérazine-1-carboxamide et 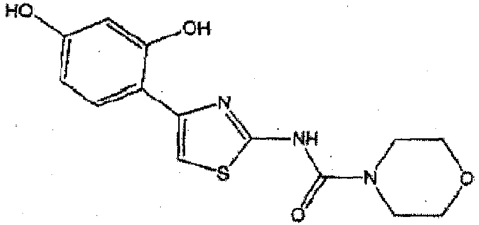 N-(4-(2,4-dihydroxyphényl)thiazol-2-yl)morpholine-4-carboxamide.

2. Hétéroalkylamidothiazoles selon la revendication 1, **caractérisés en ce qu'**ils se présentent sous la forme d'un halogénure, d'un carbonate, d'un ascorbate, d'un sulfate, d'un acétate et/ou d'un phosphate.

3. Préparations cosmétiques ou dermatologiques, ayant une teneur en un ou plusieurs hétéroalkylamidothiazoles aromatiques tels que définis dans la revendication 1 ou 2.

4. Préparations selon la revendication 3, contenant 0,000001 à 10 % en poids, notamment 0,0001 à 3 % en poids, tout particulièrement 0,001 à 1 % en poids, d'un ou de plusieurs des hétéroalkylamidothiazoles aromatiques définis dans l'une quelconque des revendications 1 ou 2, par rapport au poids total de la préparation,

5. Utilisation d'un ou de plusieurs hétéroalkylamidothiazoles aromatiques définis dans la revendication 1 ou 2, ou de préparations contenant un ou de plusieurs tels hétéroalkylamidothiazoles aromatiques, pour le traitement cosmétique et/ou la prophylaxie d'une pigmentation cutanée indésirable.

6. Hétéroalkylamidothiazoles aromatiques définis dans la revendication 1 ou 2, pour le traitement cosmétique et/ou la prophylaxie d'une pigmentation cutanée indésirable.
